# EUROPÄISCHE PATENTSCHRIFT

(11) **EP 1 963 298 B1**
(45) Veröffentlichungstag und Bekanntmachung des Hinweises auf die Patenterteilung: **02.11.2011**
(21) Anmeldenummer: 06818979.4
(22) Anmeldetag: 05.12.2006
(51) Int. Cl.: C07D 311/80, C09K 19/34

(54) **Benzochromenderivate**
Benzochromene derivatives
Dérivés de benzochromene

(30) Priorität: 22.12.2005 DE 102005062100
(43) Veröffentlichungstag der Anmeldung: 03.09.2008
(73) Patentinhaber: Merck Patent GmbH, 64293 Darmstadt (DE)
(72) Erfinder: TAUGERBECK, Andreas, 64285 Darmstadt (DE); KLASEN-MEMMER, Melanie, 67259 Heuchelheim (DE)
(86) Internationale Anmeldenummer: PCT/EP2006/011654
(87) Internationale Veröffentlichungsnummer: WO 2007/079840

(56) Entgegenhaltungen:
- DE-A1-102004 004 228
- US-A1- 2 419 934
- US-A1- 2 419 935
- COLLINS, D. J. ET AL.: "Stereochemistry of the Dihydro Compound from Lithium/Ammonia Reduction of 3-Methoxy-7,8,9,10-tetrahydro-6H-dibenzo[b ,d]pyran-6-one" AUST. J. CHEM., Bd. 49, 1996, Seiten 719-721, XP008076219
- GIROTTI, R. ET AL.: "Diels-Alder Reactions of 3-Substituted Coumarins in Water and under High-Pressure Condition. An Uncatalysed Route to Tetrahydro-6H-benzo[c]chromen-6-ones" JOURNAL OF ORGANIC CHEMISTRY, Bd. 71, 19. November 2005 (2005-11-19), Seiten 70-74, XP002424122
- PASIKOWSKA, M. ET AL.: "Theoretical studies of the Diels-Alder reaction of 1-fluoroethylene with some model dienes" THEOCHEM, Bd. 713, 13. Oktober 2004 (2004-10-13), Seiten 193-199, XP002424123
- KORONIAK, H. ET AL.: "The Influence of Fluorine Substitution on Diels-Alder Reaction of 3-Vinylcoumarin and Ethylene" POLISH JOURNAL OF CHEMISTRY, Bd. 77, Nr. 11, November 2003 (2003-11), Seiten 1523-1528, XP008076220
- GRIGG. R. ET AL.: "4-Phenylsulphinyl- and 4-Phenylsulphonyl-Coumarins as 2pi- Components in Cycloaddition Reactions" TETRAHEDRON, Bd. 45, Nr. 23, 1989, Seiten 7587-7592, XP002424124
- NARAYANA, M. ET AL.: "Phosphorus Acids in Organic Systems. III. Specific Pyrophosphoric Acid Catalysis in the Conversion of Resorcinol Dimethyl Ether to Coumarins" JOURNAL OF ORGANIC CHEMISTRY, Bd. 27, Dezember 1962 (1962-12), Seiten 4704-4706, XP002424125
- CHATTERJEA, J. N. ET AL.: "A Synthesis of O-Tetramethylbrazilin" JOURNAL OF THE INDIAN CHEMICAL SOCIETY, Bd. 51, Nr. 8, 24. April 1974 (1974-04-24), Seiten 752-754, XP008076217
- YAMAMOTO, I. ET AL.: "Halogenovinyl Sulfones 5. Synthesis of a Dibenzopyran Derivative from an Intramolecular Diels-Alder Reactionf" PHOSPHORUS, SULFUR AND SILICON, Bd. 116, 10. Mai 1996 (1996-05-10), Seiten 203-210, XP008076218
- PAREKH, M. G. ET AL.: "Studies in the Synthesis of Cyclohexenocoumarins III. Reactions of 3-Hydroxy-6-oxo-7,8,9,10-tetrahydro-6H-dib enzo[b,d]pyran" AUSTRALIAN JOURNAL OF CHEMISTRY, Bd. 23, Nr. 2, 1970, Seiten 407-412, XP008076216
- SEN, H. K. ET AL.: "Formation of Heterocyclic Compounds. III. Interaction of Cyclohexanone-2-carboxylates with Phenols" JOURNAL OF THE INDIAN CHEMICAL SOCIETY, Bd. 5, 17. Mai 1928 (1928-05-17), Seiten 467-476, XP008076226

## Beschreibung

Die vorliegende Erfindung betrifft Benzochromenderivate, bevorzugt mesogene Benzochromenderivate, insbesondere flüssigkristalline Benzochromenderivate, sowie diese Benzochromenderivate enthaltende flüssigkristalline Medien. Ferner betrifft die vorliegende Erfindung Flüssigkristallanzeigen, insbesondere mittels einer aktiven Matrix angesteuerte Flüssigkristallanzeigen (AMDs oder AM LCDs nach Englisch "active matrix addressed liquid crystal displays") und ganz insbesonders sogenannte VAN (Englisch "veritcally aligned nematics") Flüssigkristallanzeigen, einer Ausführungsform von ECB (von Englisch "electrically controlled birefringence") Flüssigkristallanzeigen, bei denen nematische Flüssigkristalle mit einer negativen dielektrischen Anisotropie (Δε) verwendet werden.

In derartigen Flüssigkristallanzeigen werden die Flüssigkristalle als Dielektrika verwendet, deren optische Eigenschaften sich bei Anlegen einer elektrischen Spannung reversibel ändern. Elektrooptische Anzeigen die Flüssigkristalle als Medien verwenden sind dem Fachmann bekannt. Diese Flüssigkristallanzeigen verwenden verschiedene elektrooptische Effekte. Die gebräuchlichsten hiervon sind der TN-Effekt (Englisch "twisted nematic"), mit einer homogenen, nahezu planaren Ausgangsorientierung des Flüssigkristalldirektors und einer um ca. 90° verdrillten nematischen Struktur), der STN-Effekt (Englisch "super-twisted nematic") und der SBE-Effekt (Englisch "supertwisted birefringence effect" mit einer 180° oder mehr verdrillten nematischen Struktur). Bei diesen und ähnlichen elektrooptischen Effekten werden flüssigkristalline Medien mit positiver dielektrischer Anisotropie (Δε) verwendet.

Neben den genannten elektrooptischen Effekten, welche Flüssigkristallmedien mit positiver dielektrischer Anisotropie benötigen, gibt es andere elektrooptische Effekte welche Flüssigkristallmedien mit negativer dielektrischer Anisotropie verwenden, wie z.B. der ECB-Effekt und seine Unterformen DAP (Englisch "deformation of aligned phases"), VAN und CSH (Englisch "color super homeotropics").

Ein elektrooptischer Effekt mit hervorragender, kleiner Blickwinkelabhängigkeit des Kontrasts verwendet axial symmetrische Micropixel (ASM von Englisch "axially symmetric micro pixel"). Bei diesem Effekt ist der Flüssigkristall jedes Pixels zylinderförmig von einem Polymermaterial umgeben. Dieser Mode eignet sich besonders zur Kombination mit der Adressierung durch Plasmakanäle. So lassen sich insbesondere großflächige PA (Englisch "plasma addressed") LCDs mit guter Blickwinkelabhängigkeit des Kontrasts realisieren.

Der in letzter Zeit verstärkt eingesetzte IPS-Effekt (Englisch "in plane switching") kann sowohl dielektrisch positive wie auch dielektrisch negative Flüssigkristallmedien verwenden, ähnlich wie auch "guest/host"-Anzeigen also Gast/Wirt-Anzeigen, die Farbstoffe je nach verwandtem Anzeigemodus entweder in dielektrisch positiven oder in dielektrisch negativen Medien einsetzen können.

Da bei Flüssigkristallanzeigen im allgemeinen, also auch bei Anzeigen nach diesen Effekten, die Betriebsspannung möglichst gering sein soll, werden Flüssigkristallmedien mit einem großen Absolutwert der dielektrischen Anisotropie eingesetzt, die in der Regel überwiegend und meist sogar weitestgehend aus Flüssigkristallverbindungen mit einer dielektrischen Anisotropie mit dem entsprechenden Vorzeichen bestehen. Also, bei dielektrisch positiven Medien aus Verbindungen mit positiver dielektrischer Anisotropie und bei dielektrisch negativen Medien aus Verbindungen mit negativer dielektrischer Anisotropie. Bei den jeweiligen Arten von Medien (dielektrisch positiv bzw. dielektrisch negativ) werden typischer Weise allenfalls nennenswerte Mengen an dielektrisch neutralen Flüssigkristallverbindungen eingesetzt. Flüssigkristallverbindungen mit dem der dielektrischen Anisotropie des Medium entgegengesetzten Vorzeichen der dielektrischen Anisotropie werden in der Regel äußerst sparsam oder gar nicht eingesetzt.

Eine Ausnahme bilden hier flüssigkristalline Medien für MIM-Anzeigen (Englisch "metal insultator metal", Simmons, J.G., Phys. Rev. 155 Nr. 3, S. 657-660 und Niwa, J.G. et al., SID 84 Digest, S. 304-307, Juni 1984) bei denen die Flüssigkristallmedien mit einer aktiven Matrix aus Dünnfilmtransistoren angesteuert werden. Bei dieser Art von Ansteuerung, welche die nicht lineare Kennlinie der Diodenschaltung ausnutzt, kann im Gegensatz zu TFT-Anzeigen kein Speicherkondensator gemeinsam mit den Elektroden der Flüssigkristallanzeigeelemente (Pixeln) aufgeladen werden. Somit ist zur Verminderung des Effekts des Spannungsabfalls während des Ansteuerzyklus ein möglichst großer Grundwert der Dielektrizitätskonstante erforderlich. Bei dielektrisch positiven Medien, wie sie z.B. bei MIM-TN-Anzeigen eingesetzt werden, muß also die Dielektrizitätskonstante senkrecht zur Molekülachse (ε_{⊥}) möglichst groß sein, da sie die Grundkapazität des Pixels bestimmt. Hierzu werden, wie z.B. in WO 93/01253, EP 0 663 502 und DE 195 21 483 beschrieben, in den dielektrisch positiven Flüssigkristallmedien, neben dielektrisch positiven Verbindungen, gleichzeitig auch Verbindungen mit negativer dielektrischer Anisotropie eingesetzt.

Eine weitere Ausnahme bilden STN-Anzeigen in denen z.B. nach DE 41 00 287 dielektrisch positive Flüssigkristallmedien mit dielektrisch negativen Flüssigkristallverbindungen eingesetzt werden um die Steilheit der elektrooptischen Kennlinie zu erhöhen.

Die Bildpunkte der Flüssigkristallanzeigen können direkt angesteuert werden, zeitsequentiell, also im Zeitmultiplexverfahren oder mittels einer Matrix von aktiven Elementen mit nichtlinearen elektrischen Kennlinien angesteuert werden.

Die bislang gebräuchlichsten AMDs verwenden diskrete aktive elektronische Schaltelemente, wie z.B. dreipolige Schaltelemente wie MOS (Englisch "metal oxide silicon") Transistoren oder Dünnfilmtransistoren (TFTs von Englisch "thin film transistors) oder Varistoren oder 2-polige Schaltelemente wie z.B. MIM (Englisch "metal insulator metal") Dioden, Ringdioden oder "back to back"-Dioden. Bei den TFTs werden verschiedene Halbleitermaterialien, überwiegend Silizium, aber auch Cadmiumselenid, verwendet. Insbesondere wird amorphes Silizium oder polykristallines Silizium verwendet.

Gemäß der vorliegenden Anmeldung sind Flüssigkristallanzeigen mit zur Flüssigkristallschicht senkrechtem elektrischen Feld und Flüssigkristallmedien mit negativer dielektrischer Anisotropie (Δε < 0) bevorzugt. Bei diesen Anzeigen ist die Randorientierung der Flüssigkristalle homeotrop. Im voll durchgeschalteten Zustand, also bei Anliegen einer entsprechend großen elektrischen Spannung, ist der Flüssigkristalldirektor parallel zur Schichtebene orientiert.

Benzochromenderivate z.B. der folgenden drei Formeln 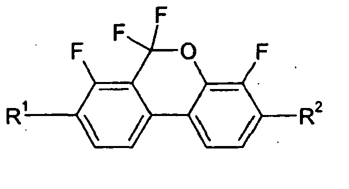 worin
R¹ und R² z.B. Alkyl bedeuten
sind aus DE 10 2002 004 228.4 und JP2005120073 bekannt. Diese Verbindungen sind durch sehr hohe Absolutwerte der dielektrischen Anisotropie gekennzeichnet und besitzen infolge der Biphenylpartialstruktur eine sehr hohe Doppelbrechung. Aufgrund der unterschiedlichen Anforderungen an die physikalischen Eigenschaften von Flüssigkristallmischungen ist es erforderlich, auch Substanzen mit niedrigerer Doppelbrechung, insbesondere z.B. für reflektive Displays, bereitzustellen. Dies gilt insbesondere für den VA-mode, da die laterale Substitution mit polaren Gruppen in der Regel an aromatischen Ringen erfolgt. Somit ist ersichtlich, dass sowohl ein Bedarf an weiteren mesogenen Verbindungen, als auch insbesondere ein Bedarf an Flüssigkristallmedien mit negativer dielektrischer Anisotropie, mit einem großen Absolutwert der dielektrischen Anisotropie, einem der jeweiligen Anwendung entsprechenden Wert der optischen Anisotropie (Δn), einer breiten nematischen Phase, guter Stabilität gegen UV, Wärme und elektrische Spannung und einer niedrigen Rotationsviskosität besteht.

Dies wird erreicht, durch Einsatz der erfindungsgemäßen mesogenen Verbindungen der Formel 1 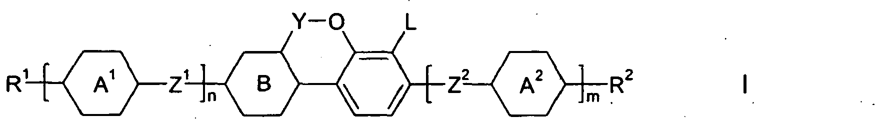 worin
- Y: CF₂
- L: H, Halogen oder CF₃ bevorzugt H, F oder Cl, besonders bevorzugt H oder F und ganz besonders bevorzugt F, 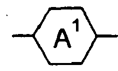
und 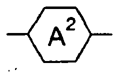 , jeweils unabhängig voneinander, und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S-ersetzt sein können,
(b) einen 1,4-Cyclohexenylenrest,
(c) einen 1,4-Phenylenrest, worin auch eine oder zwei nicht benachbarte CH-Gruppen durch N ersetzt sein können oder
(d) Naphtalin-2,6-diyl, Decahydronaphthalin-2,6-diyl und 1,2,3,4-Tetrahydronaphthalin-2,6-diyl,
(e) einen Rest ausgewählt aus der Gruppe 1,4-Bicyclo-[2,2,2]-octylen, 1,3-Bicyclo-[1,1,1]-pentylen oder Spiro-[3,3]-heptan-2,6-diyl
   wobei in
   (a) und (b) eine oder mehrere -CH₂- Gruppen, unabhängig voneinander, jeweils durch eine -CHF- oder eine -CF₂-Gruppe ersetzt sein können und in
   (c) und (d) eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können und bevorzugt 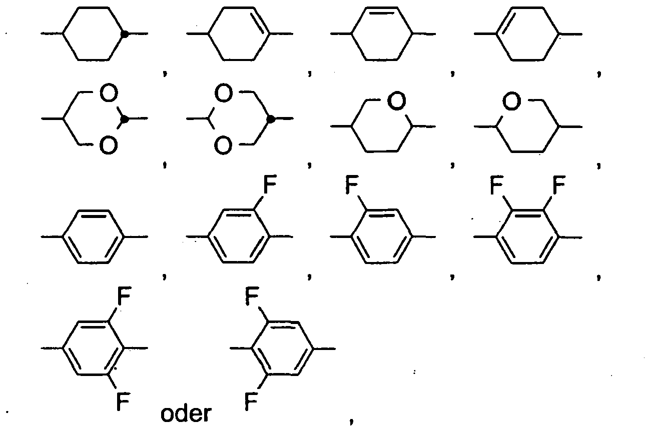 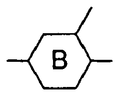 einen (1,4)-trans-Cyclohexan-1,2,4-triylrest, worin eine oder mehrere -CH₂- Gruppen, jeweils unabhängig voneinander, jeweils durch eine -CHF- oder eine -CF₂-Gruppe ersetzt sein können und die -CH< Gruppe durch eine -CF< Gruppe ersetzt sein kann, und optional eine oder zwei C-C Doppelbindungen enthalten kann wobei in diesem Fall eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können,

- R¹ und R²: , jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 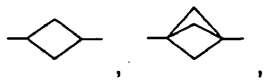 -CO-, -CO-O-, -O-CO- oder -O-CO-O- so ersetzt sein können, dass weder O- noch S-Atome direkt miteinander verknüpft sind,
bevorzugt einer von
- R¹ und R²: Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂
- Z¹ und Z²: , jeweils unabhängig voneinander, und wenn mehrfach vorhanden auch diese unabhängig voneinander, -CH₂-CH₂-, -CF₂-CF₂-, -CF₂-CH₂-, -CH₂-CF₂-, -CH=CH-, -CF=CF-, -CF=CH-, -CH=CF-, -C≡C-, -COO-, -OCO-, -CH₂O-, -OCH₂-, -CF₂O-, -OCF₂-, oder eine Kombination von zweien dieser Gruppen, wobei keine zwei O-Atome miteinander verbunden sind,
- bevorzugt: -(CH₂)₄-, -CH₂-CH₂-, -CF₂-CF₂-, -CH=CH-, -CF=CF-, -C≡C-, -CH₂O-, -CF₂O- oder eine Einfachbindung,
- besonders bevorzugt: -CH₂O-, -CH₂-CH₂-, -CF₂-CF₂-, -CF=CF-, -CF₂O- oder eine Einfachbindung und
- n und m: jeweils 0, 1 oder 2, wobei
- n + m: 0, 1, 2 oder 3, bevorzugt 0, 1 oder 2, besonders bevorzugt 0 oder 1
bedeuten.

Besonders bevorzugt sind Flüssigkristallverbindungen der Formel I-3
worin die Parameter die oben unter Formel I gegebene Bedeutung haben und
- L: bevorzugt F bedeutet.

Bevorzugt sind Verbindungen der Formel I, bevorzugt ausgewählt aus der Gruppe der Verbindungen der Formeln I-3, bei denen
die Summe n + m 0 oder 1, bevorzugt 0, beträgt.

Eine bevorzugte Ausführungsform stellen die Verbindungen der Formel I, bei denen die Summe n + m 1 beträgt und bevorzugt
- m oder n: 1 ,

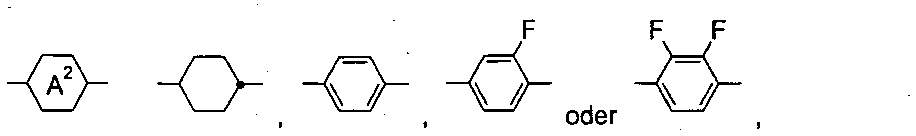

- Z²: bevorzugt -(CH₂)₄-, -CH₂-CH₂-, -CF₂-CF₂-, -CH=CH-, -CF=CF-,
-C=C-, -O-CH₂-, -O-CF₂- oder eine Einfachbindung,
besonders bevorzugt -O-CH₂-, -CH₂-CH₂-, -CF₂-CF₂-, -CF=CF-,
-O-CF₂- oder eine Einfachbindung
bedeuten und L, R¹ und R² die oben bei Formel I gegebene Bedeutung haben und L bevorzugt F bedeutet, dar.

Besonders bevorzugt sind Verbindungen der Formel I, bevorzugt ausgewählt aus der Gruppe der Verbindungen der Formeln I-3, bei denen
- n und m: beide 0 bedeuten und
L, R¹ und R² die oben bei der entsprechenden Formel gegebene Bedeutung haben und L bevorzugt F bedeutet.

Verbindungen der Formel I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verbindungen der Formel I mit S_{A}-Phasen eignen sich beispielsweise für thermisch adressierte Displays.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl, bzw. Alkoxyalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ und/oder R² einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ und/oder R² einen einfach durch CN oder CF₃ substituierten Alkyl- oder Alkenylrest bedeutet, so ist dieser Rest vorzugsweise geradkettig. Die Substitution durch CN oder CF₃ ist in beliebiger Position.

Falls R¹ und/oder R² einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verzweigte Gruppen enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ und/oder R² einen Alkylrest darstellt, in dem zwei oder mehr CH₂-Guppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl; 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl; 4,4-Bis-(ethoxycarbonyl)-butyl, 5,5-Bis-(ethoxycarbonyl)-hexyl.

Insbesondere bevorzugt sind Verbindungen der Formel I bei denen n = 0 oder 1 und m = 0 sowie R¹ Methyl, Ethyl, Propyl, Butyl, Pentyl, Vinyl, 1 E-Propenyl, 1 E-Butenyl oder 1 E-Pentenyl bedeutet so wie diese Verbindungen enthaltende Medien. Insbesondere bevorzugt von diesen Verbindungen sind die durch Alkyl substituierten Verbindungen eingesetzt.
Die Verbindungen der Formel I können aufgrund asymmetrisch substituierter Kohlenstoffatome im Ring B als Stereoisomere vorliegen. Gegenstand der Erfindung sind sämtliche Isomere, sowohl in reiner Form, als Racemat und auch als Mischung von Diastereomeren oder Enantiomeren. Optisch aktive Verbindungen der Formel I können auch als Dotierstoffe in Flüssigkristallmischungen verwendet werden.

Die Synthese der Verbindungen der Formel I (s. Schemata I bis V ) erfolgt nach den in der Literatur beschriebenen Verfahren (Houben Weyl, Methoden der Organischen Chemie, Georg Thieme Verlag, Stuttgart, New York, 4. Aufl. 1993**.** Zu Schema II s.a. DE 10 2004 004 228 (A) sowie Taugerbeck, M. Klasen-Memmer, Anmeldung Aktenzeichen 10 2004 036831.7)

In den folgenden Schemata werden die Verbindungen der Formel I kurz als Verbindungen **1** bezeichnet. Die Verbindungen **1b** und **1c** sind hierbei aus den Lactonen **1a** zugänglich. So erhält man **1b** entweder direkt durch Reduktion von **1a** mit Natriumborhydrid in Gegenwart von Bortrifluorid oder in zwei Stufen durch Reduktion von **1a** zum Diol **2** und anschließende Veretherung, z. B. durch Behandeln mit Säuren oder durch Mitsunobu-Reaktion mit Triphenylphosphin und Azodicabonsäurediethylester (siehe Schema I). 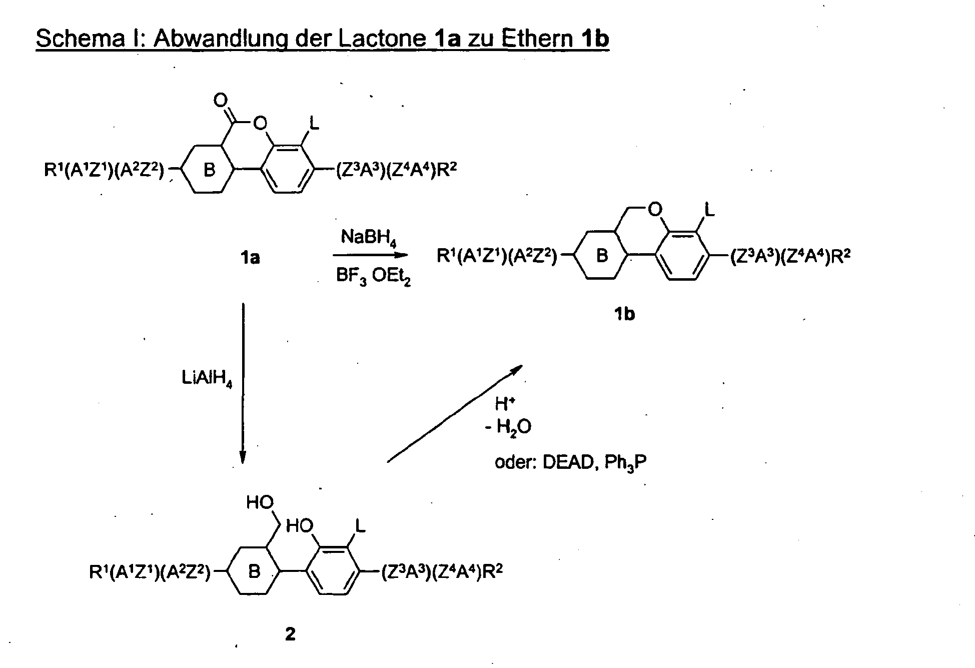 , worin, wie in den folgenden Schemata, wenn nicht explizit anders angegeben,
- R¹ und R²,: jeweils unabhängig voneinander, die oben bei Formel für R¹ bzw R² und die anderen Parameter jeweils die
entsprechenden oben bei Formel I angegebenen Bedeutungen haben.

Die Difluorether **1c** erhält man z. B. entweder durch Umsetzung der Lactone **1a** mit Lawessons Reagenz und anschließende Behandlung mit DAST oder NBS in Gegenwart von Ohlas Reagenz (W. H. Bunnelle, B. R. McKinnis, B.A. Narayanan, J. Org. Chem. 1990, 55, S. 768-770) oder in Analogie zu dem in A. Taugerbeck, M. Klasen-Memmer, Anmeldung Aktenzeichen 10 2004 036831.7 beschriebenen Verfahren durch Fluorodesulfurierung von Dithioorthoestern vom Typ **4** mit einem Oxidationsmittel wie z.B. Brom, NBS, DBH u.a. in Gegenwart einer Fluoridionenquelle wie HF-Pyridinkomplex, Triethylamintrishydrogenfluorid, etc. (siehe Schema II). 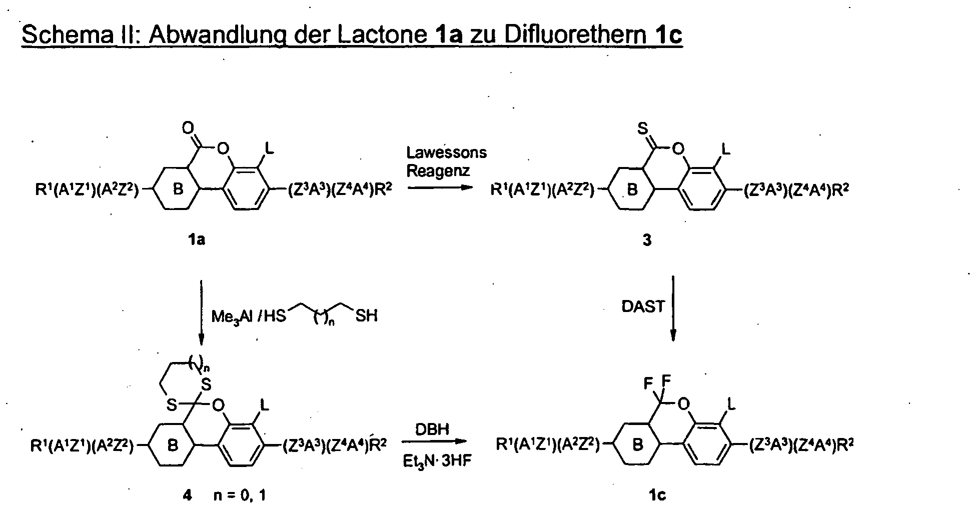 worin n = 0,1.

Die Herstellung der Lactone **1a** erfolgt gemäß S. Sethna, R. Phadke, Org. React. 1953, 7, S. 1 durch Pechmann-Kondensation von Phenolderivaten oder Resorcinen mit β-Ketoestern vom Typ **6** (V. H. Wallingford, A. H. Homeyer, D. M. Jones, J. Am. Chem. Soc. 1941, 63, S. 2252-2254) und nachfolgende Hydrierung (Schema 3).

Eine alternative Hydrierung der Verbindungen 7 mit Lithium in Ammoniak ist in D. J. Collins, A. G. Ghingran, S. B. Rutschmann, Aust. J. Chem. 1989, 42, S. 1769-1784 beschrieben.

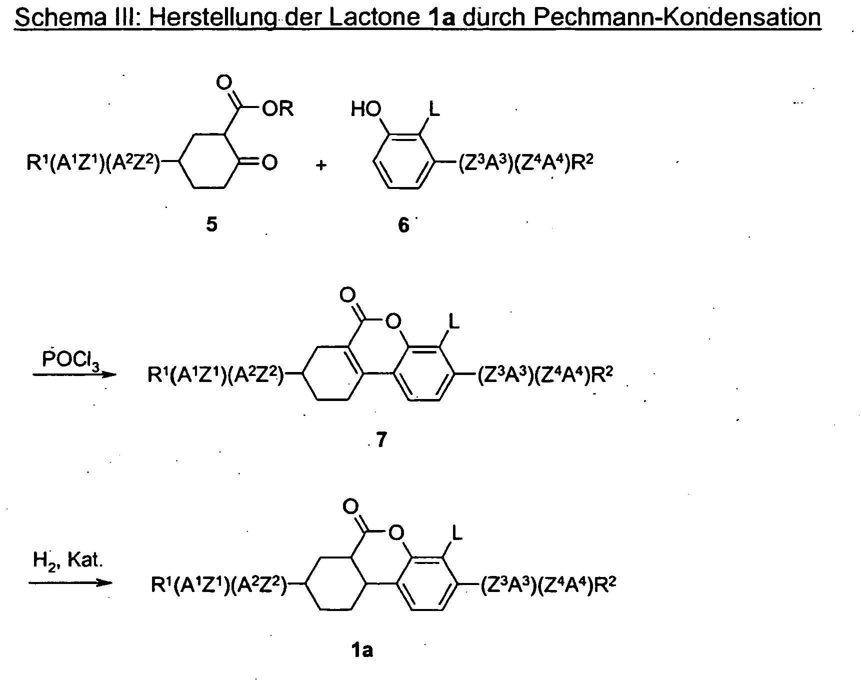

Die Verbindungen **7** sind auch nach P. Sellès, U. Mueller, Org. Lett. 2004, 6, S. 277-279 durch Suzuki-Kupplung aus Enoltriflaten **8** erhältlich (siehe Schema IV). Die Verbindungen **8** können aus den oben beschriebenen Ketoestem **5** durch Behandeln mit Trifluormethansulfonsäureanhydrid in Gegenwart einer Base wie z.B. Collidin erhalten werden (E. Piers, H. L. A.Tse, Tetrahedron Lett. 1984, 25, 3155-3158). Die Synthese der Boronsäuren **9** kann z. B. in Analogie zu A. Taugerbeck, M. Klasen-Memmer, DE102004004228 durch Umsetzung der entsprechenden Arylbromide durch Brom-Lithium-Austausch und anschließende Reaktion mit Trimethylborat erfolgen.

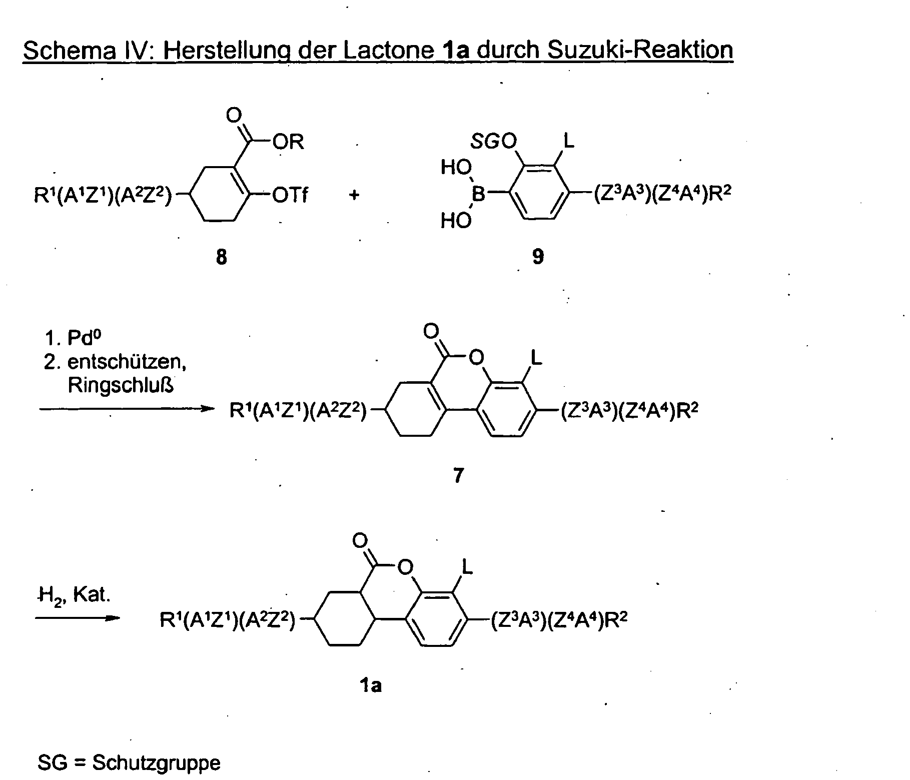

Die Verbindungen **1a** fallen nach Hydrierung als Isomerengemisch an, das duch die üblichen Verfahren, Kristallisation und/oder Chromatographie getrennt werden kann. Verbindungen mit 6a*R**,8*R**,10a*S**-Konfiguration können gemäß Schema V in zwei zusätzlichen Syntheseschritten oder nach D. J. Collins, A. G. Ghingran, S. B. Rutschmann, Aust. J. Chem. 1989, 42, S. 1769-1784 durch basenkatalysierte Isomerisierung erhalten werden, wobei es vorteilhaft sein kann, gemäß J. M. Fevig et al., Bioorg. Med. Chem. Lett. 1996, 6, S. 295-300 den Lactonring zunächst durch Verseifen zu öffnen und nach erfolgter basenkatalysierter Isomerisierung wieder zu schließen. 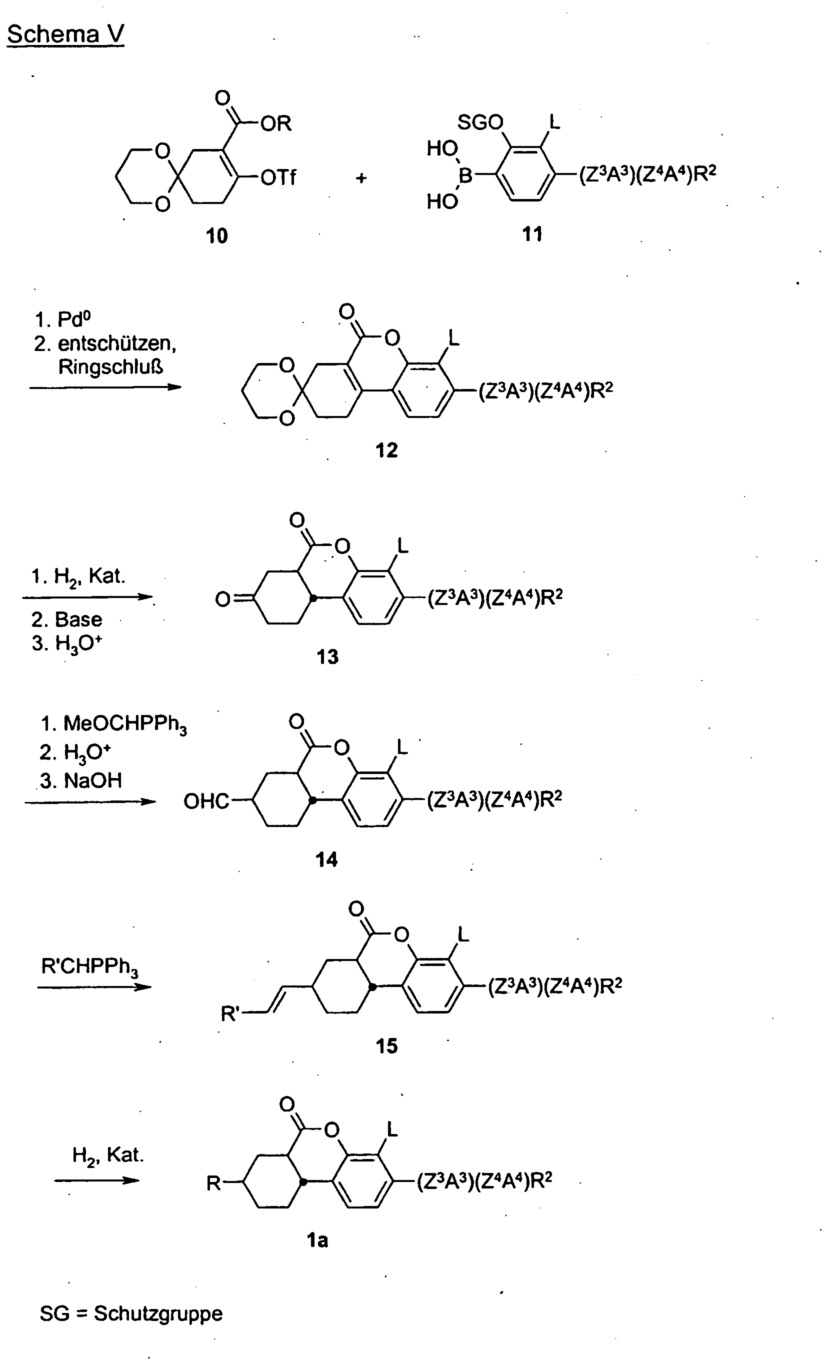 Im Folgenden werden Beispiele für Strukturen bevorzugter Verbindungen der Formel I gegeben, worin R und R' die jeweilige unter Formel I für R¹ bzw. R² gegebene Bedeutung haben.

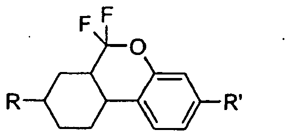

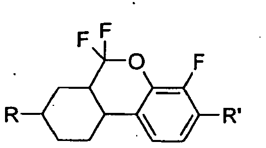

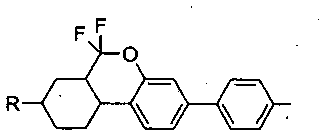

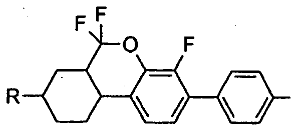

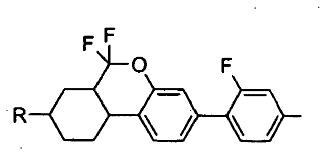

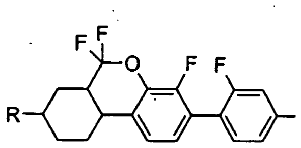

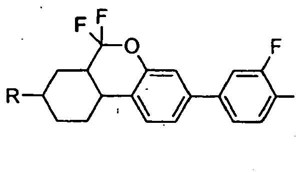

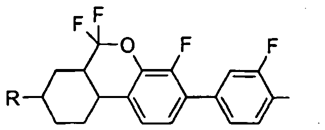

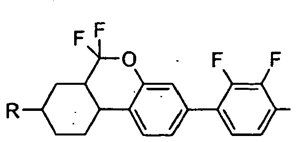

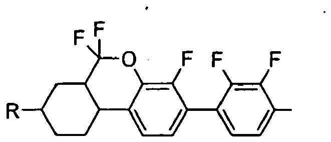

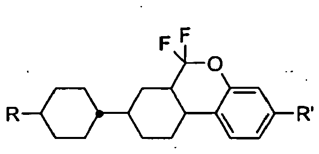

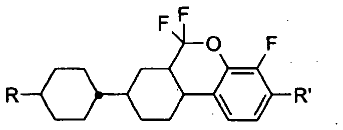

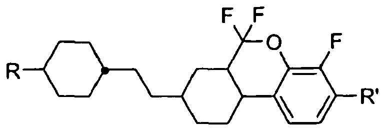

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Gegenstand der vorliegenden Erfindung sind auch Flüssigkristallmedien, die eine oder mehrere Verbindung(en) der Formel I enthalten.

In einer bevorzugten Ausführungsform enthalten die Flüssigkristallmedien gemäß der vorliegenden Erfindung
a) eine oder mehrere dielektrisch negative Verbindung(en) der Formel I 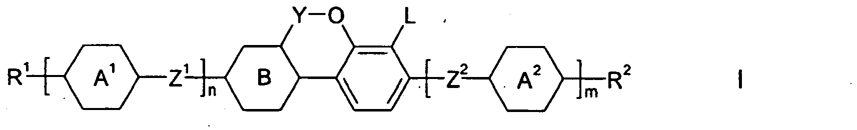 worin
   - Y: CF₂
   - L: H, Halogen oder CF₃, bevorzugt H, F oder Cl, besonders bevorzugt H oder F und ganz besonders bevorzugt F, 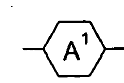
   und 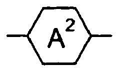 , jeweils unabhängig voneinander, und wenn mehrfach vorhanden auch diese unabhängig voneinander,
   (a) einen trans-1,4-Cyclohexylenrest, worin auch eine oder zwei nicht benachbarte CH₂-Gruppen durch -O- und/oder -S-ersetzt sein können,
   (b) einen 1,4-Cyclohexenylenrest,
   (c) einen 1,4-Phenylenrest, worin auch eine oder zwei nicht benachbarte CH-Gruppen durch N ersetzt sein können oder
   (d) Naphtalin-2,6-diyl, Decahydronaphthalin-2,6-diyl und 1,2,3,4-Tetrahydronaphthalin-2,6-diyl,
   (e) einen Rest ausgewählt aus der Gruppe 1,4-Bicyclo-[2,2,2]-octylen, 1,3-Bicyclo-[1,1,1]-pentylen oder Spiro-[3,3]-heptan-2,6-diyl,
      wobei in
      (a) und (b) eine oder mehrere -CH₂- Gruppen, unabhängig vonenander, jeweils durch eine -CHF- oder eine -CF₂-Gruppe ersetzt sein können und in
      (c) und (d) eine oder mehrere -CH= Gruppen, unabhängig vonenander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können und bevorzugt 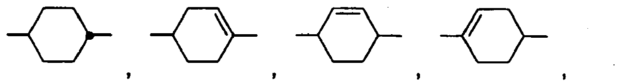 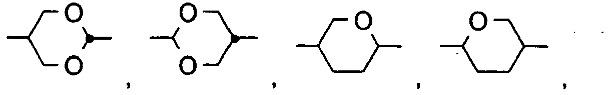 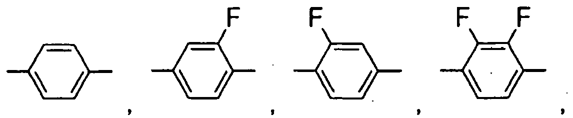 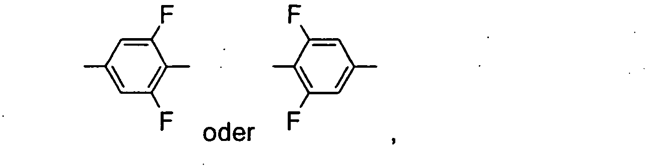 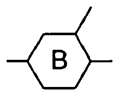
      einen (1,4)-trans-Cyclohexan 1,2,4-triylrest, worin eine oder mehrere -CH₂- Gruppen, jeweils unabhängig voneinander, jeweils durch eine -CHF- oder eine -CF₂-Gruppe ersetzt sein können und die -CH< Gruppe durch eine -CF< Gruppe ersetzt sein kann, und optional eine oder zwei C-C Doppelbindungen enthalten kann wobei in diesem Fall eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können,
      - R¹ und R²: , jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 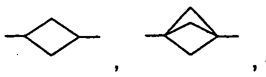 -CO-, -CO-O-, -O-CO- oder -O-CO-O- so ersetzt sein können, dass weder O- noch S-Atome direkt miteinander verknüpft sind,
      bevorzugt einer von
      - R¹ und R²: Alkyl und Alkoxy mit 1 bis 12 C-Atomen, Alkoxyalkyl, Alkenyl oder Alkenyloxy mit 2 bis 12 C-Atomen und der andere, unabhängig vom ersten, ebenfalls Alkyl und Alkoxy mit 1 bis 12 C-Atomen, Alkoxyalkyl, Alkenyl oder Alkenyloxy mit 2 bis 12 C-Atomen oder auch F, Cl, Br, -CN, -SCN, -SF₅, -CF₃, -CHF₂, -CH₂F, -OCF₃ oder -OCHF₂
      - Z¹ und Z²: , jeweils unabhängig voneinander, und wenn mehrfach vorhanden auch diese unabhängig voneinander, -CH₂-CH₂-, -CF₂-CF₂-, -CF₂-CH₂-, -CH₂-CF₂-, -CH=CH-, -CF=CF-, -CF=CH-, -CH=CF-, -C≡C-, -COO-, -OCO-, -CH₂O-, -OCH₂-, -CF₂O-, -OCF₂-, oder eine Kombination von zweien dieser Gruppen, wobei keine zwei O-Atome miteinander verbunden sind,
      - bevorzugt: -(CH₂)₄-, -CH₂-CH₂-, -CF₂-CF₂-, -CH=CH-, -CF=CF-, -C≡C-, -CH₂O-, -CF₂O- oder eine Einfachbindung,
      - besonders bevorzugt: -CH₂O-, -CH₂-CH₂-, -CF₂-CF₂-, -CF=CF-, -CF₂O- oder eine Einfachbindung und
      bedeuten,
      - n und m: jeweils 0, 1 oder 2, wobei
      - n + m: 0, 1, 2 oder 3, bevorzugt 0, 1 oder 2, besonders bevorzugt 0 oder 1
b) eine oder mehrere dielektrisch negative Verbindung(en) der Formel II 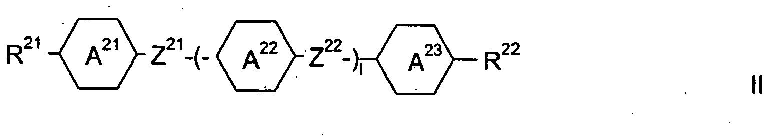 worin
   - R²¹ und R²²: jeweils unabhängig voneinander die oben bei Formel I für R¹ gegebene Bedeutung haben,
   - Z²¹ und Z²²: jeweils unabhängig voneinander die oben bei Formel I für Z¹ gegebene Bedeutung haben,
   mindestens einer der vorhandenen Ringe 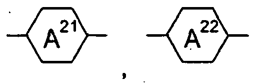 und 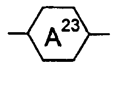 bevorzugt 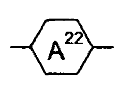 und 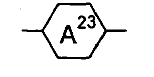 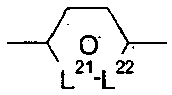 und die anderen, jeweils unabhängig
   voneinander, 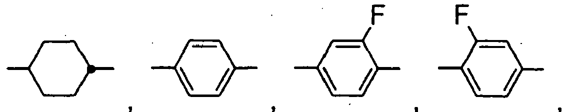 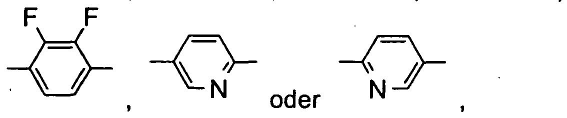 bevorzugt 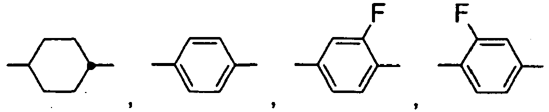 oder 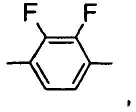 besonders bevorzugt 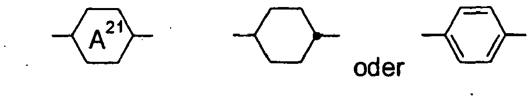 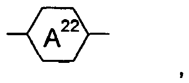 wenn vorhanden, 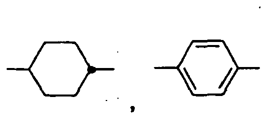 oder 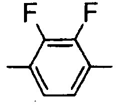
   - L²¹ und L²²: beide C-F oder einer von beiden N und der andere C-F, bevorzugt beide C-F und
   - I: 0, 1 oder 2, bevorzugt 0 oder 1
   bedeuten;
   und optional
c) eine oder mehrere dielektrish neutrale Verbindung der Formel III 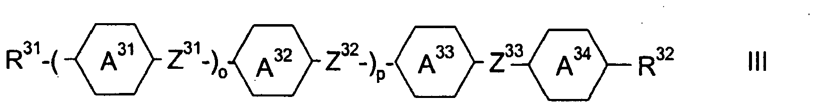 worin
   - R³¹ und R³²: jeweils unabhängig voneinander die oben bei Formel I für R¹ gegebene Bedeutung besitzen und
   - Z³¹, Z³² und Z³³: jeweils unabhängig voneinander -CH₂CH₂-, -CH=CH-, -COO- oder eine Einfachbindung 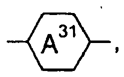 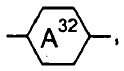 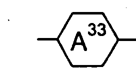
   und 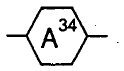 jeweils unabhängig voneinander 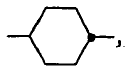 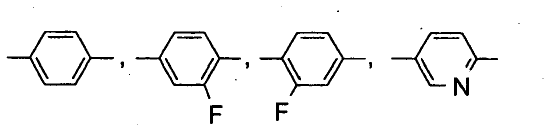 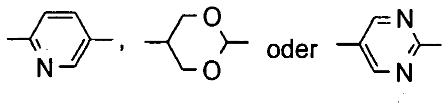 und
   - o und p: unabhängig voneinander 0 oder 1
   bevorzugt jedoch
   - R³¹ und R³²: jeweils unabhängig voneinander Alkyl oder Alkoxy mit 1-5 C-Atomen oder Alkenyl mit 2-5 C-Atomen, 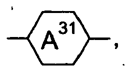 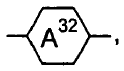 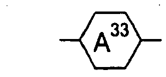
   und 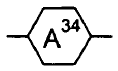 jeweils unabhängig voneinander 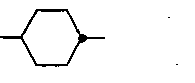 oder 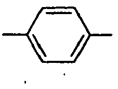 und ganz besonders bevorzugt mindestens zwei dieser Ringe 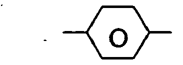 und/oder 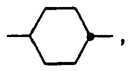 wobei ganz besonders bevorzugt zwei benachbarte Ringe direkt verknüpft sind und zwar bevorzugt 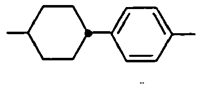 oder 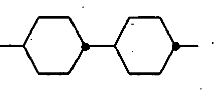
bedeuten, wobei bei dem Phenylenring ein oder mehrere H-Atome, unabhängig voneinander durch F oder CN, bevorzugt durch F und eine oder zwei nicht benacbarte CH₂-Gruppen des Cyclohexylenrings bzw. eines der Cyclohexylenringe durch O-Atome ersetzt sein können.

Bevorzugt enthalten die Flüssigkristallmedien eine oder mehrere Verbindungen der Formel I die keine Biphenyleinheit enthalten.

Besonders bevorzugt enthalten die Flüssigkristallmedien eine oder mehrere Verbindungen der Formel I verknüpft sind und zwar bevorzugt 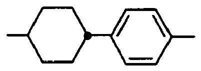 oder 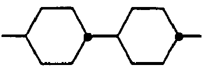 bedeuten, wobei bei dem Phenylenring ein oder mehrere H-Atome, unabhängig voneinander durch F oder CN, bevorzugt durch F und eine oder zwei nicht benacbarte CH₂-Gruppen des Cyclohexylenrings bzw. eines der Cyclohexylenringe durch O-Atome ersetzt sein können.

In einer bevorzugten Ausführungsform, die mit den gerade beschriebenen Ausführungsformen identisch sein kann, enthalten die Flüssigkristallmedien eine oder mehrere Verbindungen ausgewählt aus der Gruppe der Verbindungen ausgewählt aus der Gruppe der Verbindungen der Formel I-3.

Bevorzugt enthält das Flüssigkristallmedium eine oder mehrere Verbindungen ausgewählt aus der Gruppe der Verbindungen der Formeln II-1 bis II-3 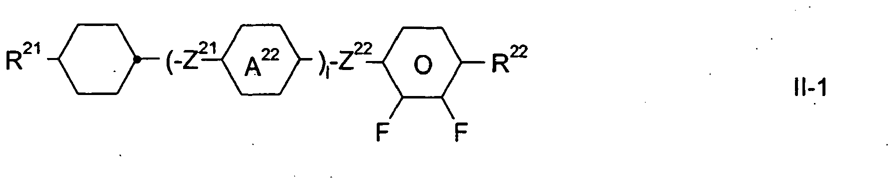 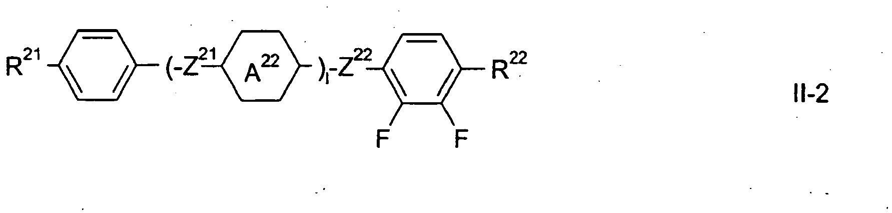 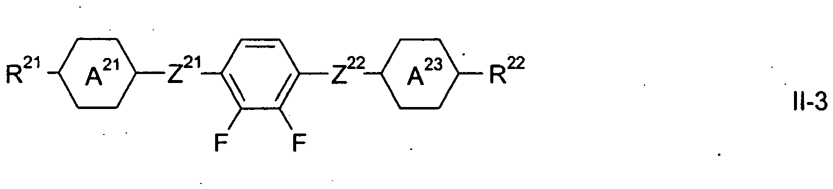 worin R²¹, R²², Z¹², Z²², 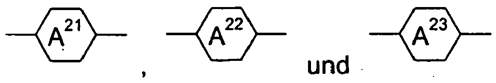 und I jeweils die oben bei Formel II gegebene Bedeutung besitzen. Bevorzugt ist R²¹ Alkyl, bevorzugt mit 1-5 C-Atomen, R²¹ Akyl oder Alkoxy, bevorzugt jeweils mit 1 bis 5 C-Atomen, und Z²² sowie Z²¹, wenn vorhanden, eine Einfachbindung.

Besonders bevorzugt enthält das Flüssigkristallmedium eine oder mehrere Verbindungen ausgewählt aus der Gruppe der Verbindungen der Formeln III-1 bis III-3: 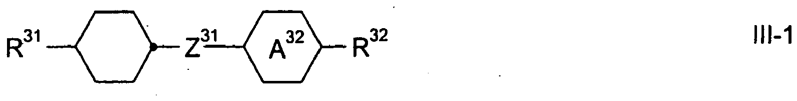 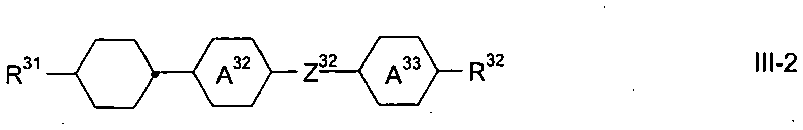 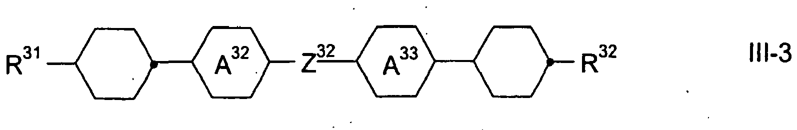 worin R³¹, R³², Z³¹, Z³², 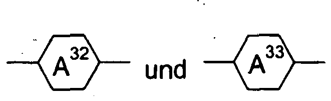 jeweils die oben bei Formel III angegebene Bedeutung haben.

Insbesondere bevorzugt enthält das Flüssigkristallmedium eine oder mehrere Verbindungen ausgewählt aus der Gruppe der Verbindungen der Formeln III-1a bis III-1d, III-1e, 111-2a bis III-2g, III-3a bis III-3d und III-4a: 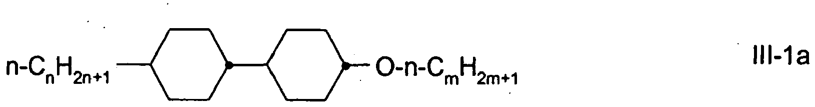 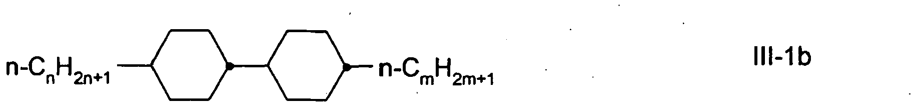 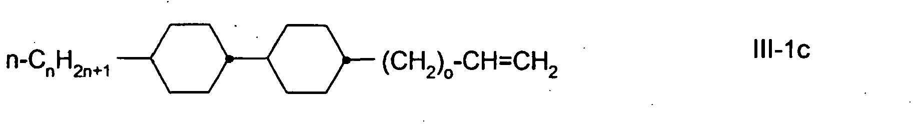 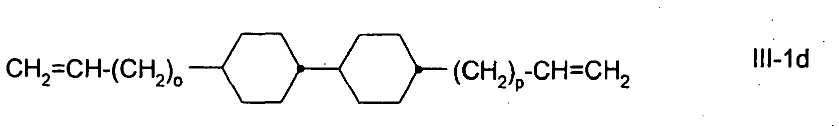 worin n und m jeweils unabhängig voneinander 1 bis 5 und o und p jeweils sowohl davon als auch voneinander unabhängig 0 bis 3 bedeuten, 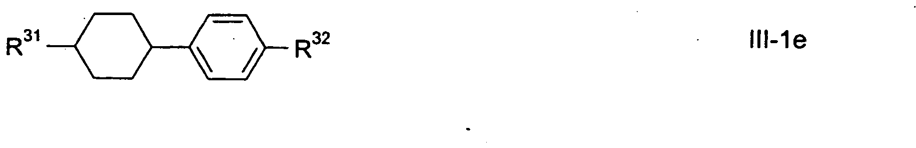 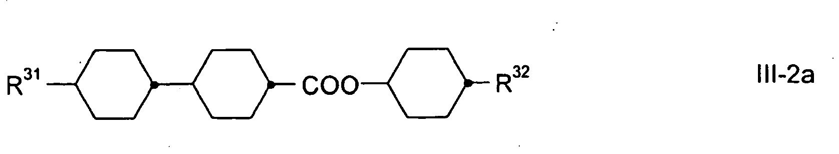 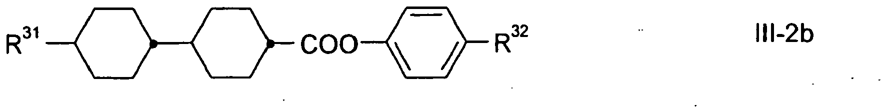 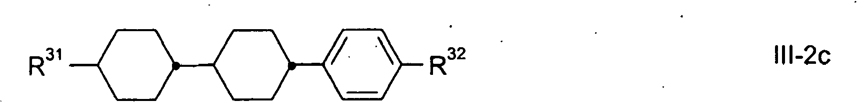 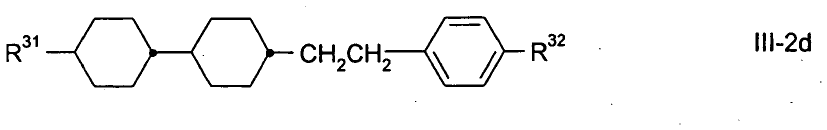 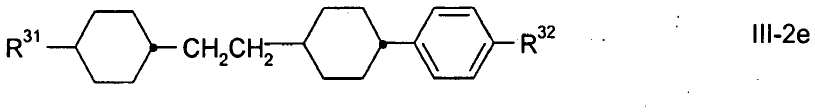 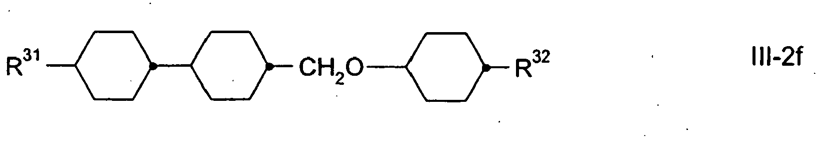 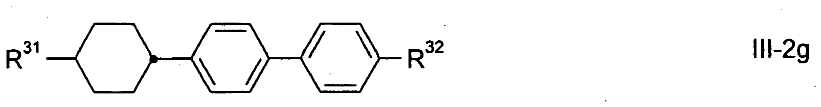 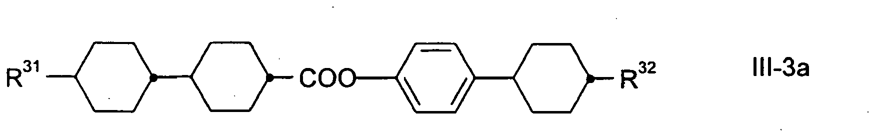 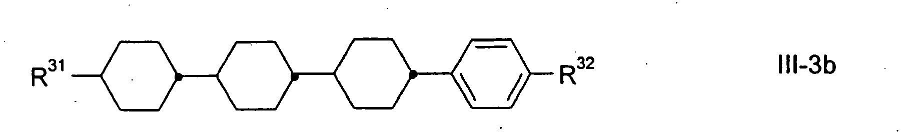 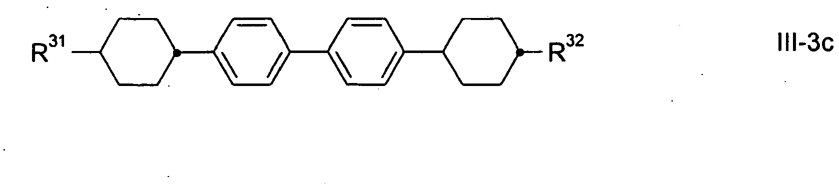 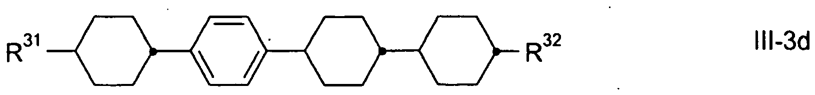 worin R³¹ und R³³ jeweils die oben unter Formel III, bevorzugt die unter Formel III-1, angegebene Bedeutung besitzen und die Phenylringe, insbesondere bei den Verbindungen III-2g und III-3c optional fluoriert sein können, jedoch nicht so, dass die Verbindungen mit denen der Formel II und ihren Unterformeln identisch sind. Bevorzugt ist R³¹ n-Alkyl mit 1 bis 5 C-Atomen, insbesondere bevorzugt mit 1 bis 3 C-Atomen und R³² n-Alkyl oder n-Alkoxy mit 1 bis 5 C-Atomen oder Alkenyl mit 2 bis 5 C-Atomen. Hiervon sind insbesondere Verbindungen der Formeln III-1a bis III-1d bevorzugt.

Bevorzugte fluorierte Verbindungen der Formeln III-2g und III-3c sind die Verbindungen der Formeln III-2g' und III-3c' 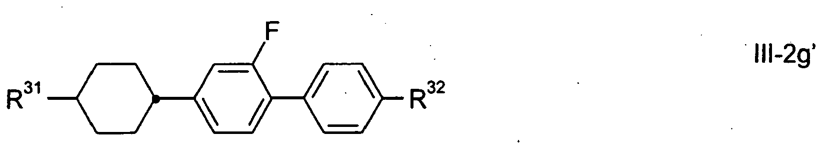 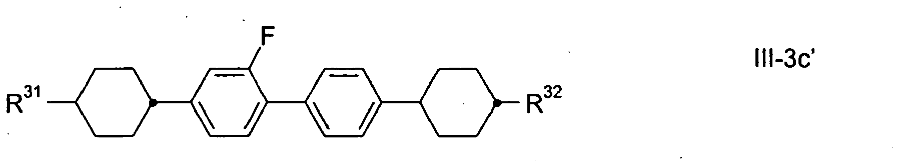 worin R³¹ und R³³ jeweils die oben unter Formel III, bevorzugt die unter Formel III-2g, bzw. III-3c angegebene Bedeutung haben.

In der vorliegenden Anmeldung bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Hierbei bedeutet der Begriff eine nematische Phase aufweisen einerseits, daß bei tiefen Temperaturen bei der entsprechenden Temperatur keine smektische Phase und keine Kristallisation beobachtet wird und andererseits, daß beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, einer der elektrooptischen Anwendung entsprechenden Schichtdicke, für mindestens 100 Stunden überprüft. Bei hohen Temperaturen wird der Klärpunkt nach üblichen Methoden in Kapillaren gemessen.

Ferner sind die erfindungsgemäßen Flüssigkristallmedien durch niedrige optische Anisotropien gekennzeichnet.

Der Ausdruck "Alkyl" umfaßt vorzugsweise geradkettige und verzweigte Alkylgruppen mit 1 bis 7 Kohlenstoffatomen, insbesondere die geradkettigen Gruppen Methyl, Ethyl, Propyl, Butyl, Pentyl, Hexyl und Heptyl. Gruppen mit 2 bis 5 Kohlenstoffatomen sind im allgemeinen bevorzugt.

Der Ausdruck "Alkenyl" umfaßt vorzugsweise geradkettige und verzweigte Alkenylgruppen mit 2 bis 7 Kohlenstoffatomen, insbesondere die geradkettigen Gruppen. Besonders bevorzugte Alkenylgruppen sind C₂ bis C₇-1 E-Alkenyl, C₄ bis C₇-3E-Alkenyl, C₅ bis C₇-4-Alkenyl, C₆ bis C₇-5-Alkenyl und C₇ 6-Alkenyl, insbesondere C₂ bis C₇-1 E-Alkenyl, C₄ bis C₇-3E-Alkenyl und C₅ bis C₇-4-Alkenyl. Beispiele weiterer bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n 1 und m 1 bis 6.

Verbindungen mir einer Vinyl-Endgruppe und Verbindungen mit einer Methyl-Endgruppe haben eine geringe Rotationsviskosität.

In der vorliegenden Anmeldung bedeuten die Begriffe dielektrisch positive Verbindungen solche Verbindungen mit einem Δε > 1,5, dielektrisch neutrale Verbindungen solche mit -1,5 ≤ Δε ≤ 1,5 und dielektrisch negative Verbindungen solche mit Δε < -1,5. Hierbei wird die dielektrische Anisotropie der Verbindungen bestimmt indem 10 % der Verbindungen in einem flüssigkristallinen Host gelöst werden und von dieser Mischung die Kapazität in mindestens jeweils einer Testzelle mit ca. 20 µm Schichtdicke mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristallmischung.

Als Wirtsmischung für die Bestimmung der anwendungsrelevanten physikalischen Parameter wird ZLI-4792, von Merck KGaA, Deutschland, verwendet. Als Ausnahme wird bei der Bestimmung der dielektrischen Anisotropie von dielektrisch negativen Verbindungen ZLI-2857, ebenfalls von Merck KGaA, Deutschland, verwendet. Aus der Änderung der Eigenschaften, z.B. der Dielektrizitätskonstanten, der Wirtsmischung nach Zugabe der zu untersuchenden Verbindung und Extrapolation auf 100 % der eingesetzten Verbindung werden die Werte für die jeweilige zu untersuchende Verbindung erhalten.

Die eingesetzte Konzentration der zu untersuchenden Verbindung beträgt 10 %. Ist die Löslichkeit der zu untersuchenden Verbindung hierzu nicht ausreichend wird ausnahmsweise die eingesetzte Konzentration solange halbiert, also auf 5 %, 2,5 % usw. verringert, bis die Löstichkeitsgrenze unterschritten ist.

Der Begriff Schwellenspannung bezieht sich üblicherweise auf die optische Schwelle für 10 % relativen Kontrast (V₁₀). In Bezug auf die Flüssigkristallmischungen mit negativer dielektrischer Anisotropie, wird der Begriff Schwellenspannung in der vorliegenden Anmeldung jedoch für die kapazitive Schwellenspannung (V₀), auch Freedericksz-Schwelle genannt, verwendet, sofern nicht explizit anders angegeben.

Alle Konzentrationen in dieser Anmeldung sind, soweit nicht explizit anders vermerkt, in Massenprozent angegeben und beziehen sich auf die entsprechende Gesamtmischung. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. Δn wird bei 589 nm und Δε bei 1 kHz bestimmt.

Bei den Flüssigkristallmedien mit negativer dielektrischer Anisotropie wurde die Schwellenspannung als kapazitive Schwellung V₀ in Zellen mit durch Lecithin homeotrop orientierter Flüssigkristallschicht bestimmt.

Die erfindungsgemäßen Flüssigkristallmedien können bei Bedarf auch weitere Zusatzstoffe und gegebenenfalls auch chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentrationen der einzelnen eingesetzten Verbindungen betragen jeweils bevorzugt 0,1 bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den Komponenten gelöst, die den Hauptbestandteil ausmachen, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus sogenannten "multi bottle" Systemen herzustellen.

Mittels geeigneter Zusatzstoffe können die erfindungsgemäßen Flüssigkristallphasen derart modifiziert werden, dass sie in jeder bisher bekannt gewordenen Art von Anzeige und insbesondere von ECB-Anzeigen, sowie IPS-Anzeigen einsetzbar sind.

Die nachstehenden Beispiele dienen zur Veranschaulichung der Erfindung, ohne sie zu beschränken. In den Beispielen sind der Schmelzpunkt T(C,N), der Übergang von der smektischen (S) zur nematischen (N) Phase T(S,N) und Klärpunkt T(N,I) einer Flüssigkristallsubstanz in Grad Celsius angegeben. Die verschiedenen smektischen Phasen werden durch entsprechende Suffixe gekennzeichnet.

Die Prozentangaben sind, soweit nicht explizit anders gekennzeichnet, vor- und nachstehend Massenprozente und die physikalischen Eigenschaften sind die Werte bei 20 °C, sofern nicht explizit anders angegeben.

Alle angegebenen Werte für Temperaturen in dieser Anmeldung sind °C und alle Temperaturdifferenzen entsprechend Differenzgrad, sofern nicht explizit anders angegeben.

Bei den Synthesebeispielen und -schemata bedeuten:
- DAST: Diethylaminoschwefeltrifluorid,
- DBH: Dibromdimethylhydantoin
- DEAD: Diethylazodicarboxylat
- MBT: MBT-Ether, Methyl-tert-butylether und

- NBS: N-Bromsuccinimid
- THF: Tetrahydrofuran.

In der vorliegenden Anmeldung und in den folgenden Beispielen sind die Strukturen der Flüssigkristallverbindungen durch Abkürzungen, auch Acronyme genannt, angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ , L² und L³:

| Code für R¹, R², L¹, L², L³ | R¹ | R² | L¹ | L² | L³ |
|---|---|---|---|---|---|
| nm | CₙH₂ₙ₊₁ | CₘH₂ₘ₊₁ | H | H | H |
| nOm | CₙH₂ₙ₊₁ | OCₘH₂ₘ₊₁ | H | H | H |
| nO.m | OCₙH₂ₙ₊₁ | CₘH₂ₘ₊₁ | H | H | H |
| nmFF | CₙH₂ₙ₊₁ | CₘH₂ₘ₊₁ | F | H | F |
| nOmFF | CₙH₂ₙ₊₁ | OCₘH₂ₘ₊₁ | F | H | F |
| nO.mFF | OCₙH₂ₙ₊₁ | CₘH₂ₘ₊₁ | F | H | F |
| nO.OmFF | OCₙH₂ₙ₊₁ | OCₘH₂ₘ₊₁ | F | H | F |
| n | CₙH₂ₙ₊₁ | CN | H | H | H |
| nN.F | CₙH₂ₙ₊₁ | CN | F | H | H |
| nN.F.F | CₙH₂ₙ₊₁ | CN | F | F | H |
| nF | CₙH₂ₙ₊₁ | F | H | H | H |
| nF.F | CₙH₂ₙ₊₁ | F | F | H | H |
| nF.F.F | CₙH₂ₙ₊₁ | F | F | F | H |
| nCl | CₙH₂ₙ₊₁ | Cl | H | H | H |
| nCl.F | CₙH₂ₙ₊₁ | Cl | F | H | H |
| nCl.F.F | CₙH₂ₙ₊₁ | Cl | F | F | H |
| nmF | CₙH₂ₙ₊₁ | CₘH₂ₘ₊₁ | F | H | H |
| nCF₃ | CₙH₂ₙ₊₁ | CF₃ | H | H | H |
| nOCF₃ | CₙH₂ₙ₊₁ | OCF₃ | H | H | H |
| nOCF₃.F | CₙH₂ₙ₊₁ | OCF₃ | F | H | H |
| nOCF₃.F.F | CₙH₂ₙ₊₁ | OCF₃ | F | F | H |
| nOCF₂ | CₙH₂ₙ₊₁ | OCHF₂ | H | H | H |
| nOCF₂.F.F | CₙH₂ₙ₊₁ | OCHF₂ | F | F | H |
| nS | CₙH₂ₙ₊₁ | NCS | H | H | H |
| rVsN | CᵣH₂ᵣ₊₁-CH=CH-CₛH₂ₛ- | CN | H | H | H |
| nEsN | CᵣH₂ᵣ₊₁-O-CₛH₂ₛ- | CN | H | H | H |
| nAm | CₙH₂ₙ₊₁ | COOCₘH₂ₘ₊₁ | H | H | H |
| nF.Cl | CₙH₂ₙ₊₁ | F | Cl | H | H |

**Tabelle A:**

| | |
|---|---|
| 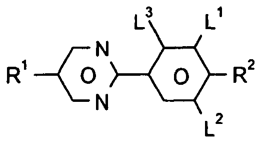 **PYP** | 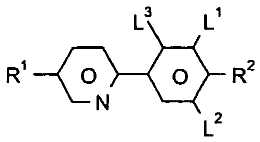 **PYRP** |
| 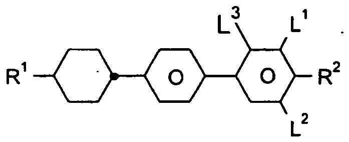 **BCH** | 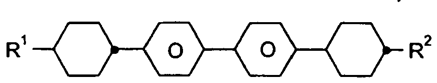 **CBC** |
| 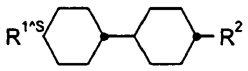 **CCH** | 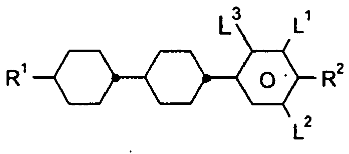 **CCP** |
| 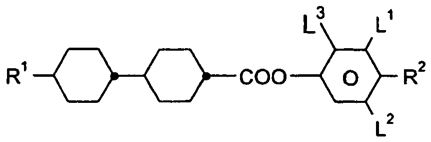 **CP** | 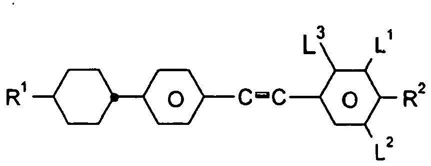 **CPTP** |
| 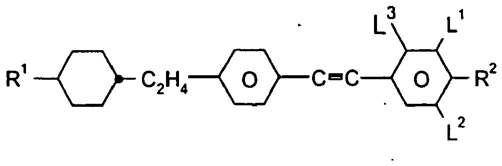 **CEPTP** | |
| 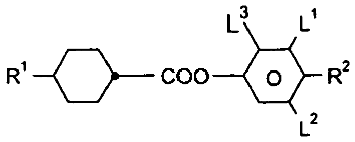 **D** | 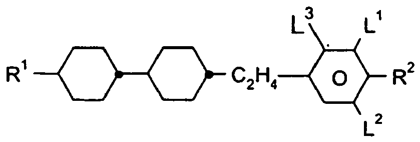 **ECCP** |
| 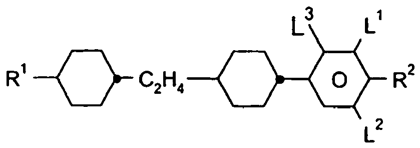 **CECP** | 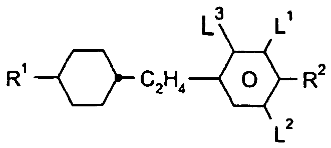 **EPCH** |
| 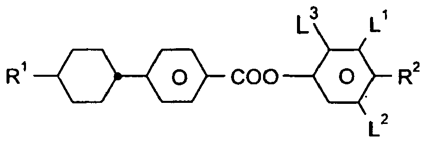 **HP** | 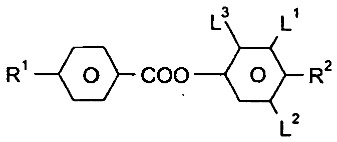 **ME** |
| 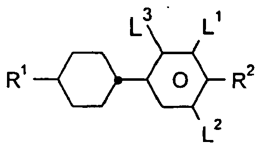 **PCH** | 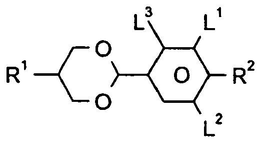 **PDX** |
| 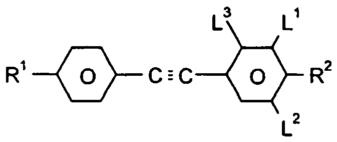 **PTP** | 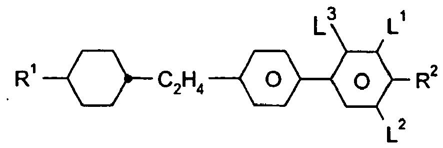 **BECH** |
| 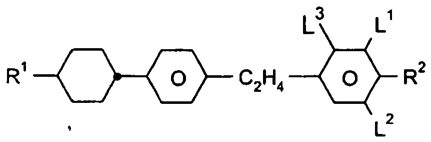 **EBCH** | 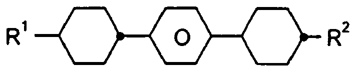 **CPC** |
| 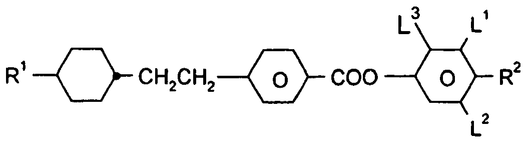 **EHP** | |
| 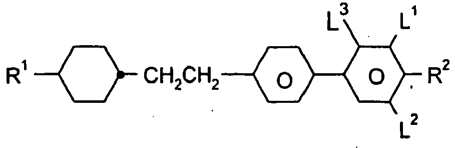 **BEP** | |
| 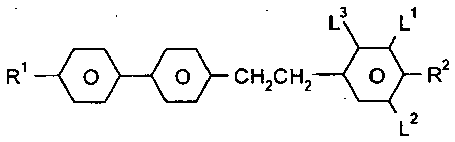 **ET** | |

**Tabelle B:**

| | |
|---|---|
| 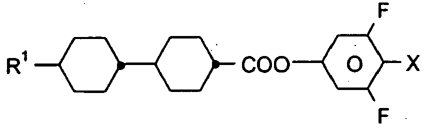 **CCZU-n-X** (X = F, Cl, -OCF3 = "OT") | 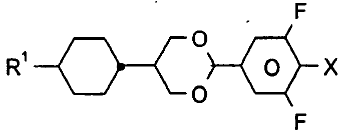 **CDU-n-X** (X = F, Cl, -OCF3 = "OT") |
| 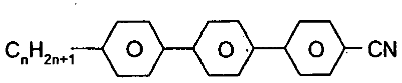 **T3n** | 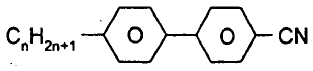 **K3n** |
| 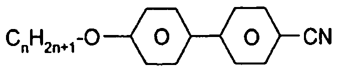 **M3n** | 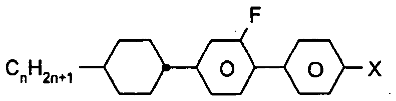 **CGP-n-X** (X = F, Cl, -OCF3 = "OT") |
| 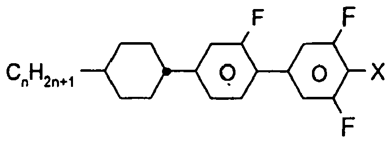 **CGU-n-X** (X = F, Cl, -OCF3 = "OT") | 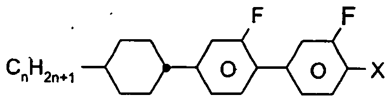 **CGG-n-X** (X = F, Cl, -OCF3 = "OT") |
| 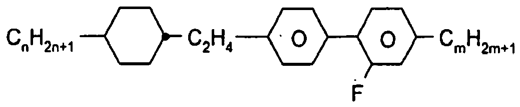 **Inm** | |
| 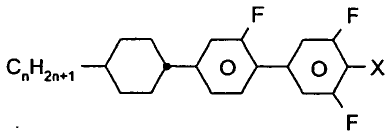 **CGU-n-X** (X = F, Cl, -OCF3 = "OT") | |
| 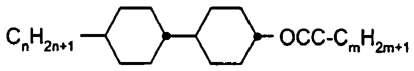 **C-nm** | 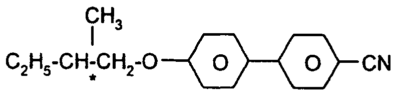 **C15** |
| 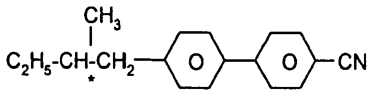 **CB15** | |
| 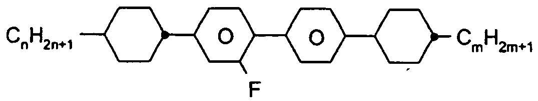 **CBC-nmF** | |
| 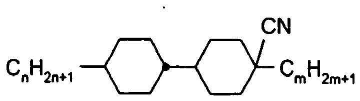 **CCN-nm** | 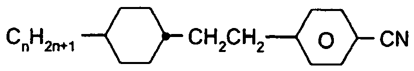 **G3n** |
| 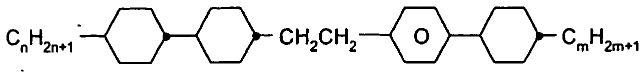 **CCEPC-nm** | |
| 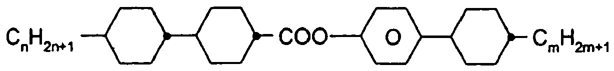 **CCPC-nm** | |
| 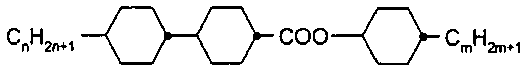 **CH-nm** | |
| 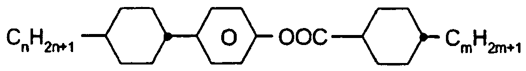 **HD-nm** | |
| 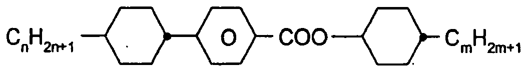 **HH-nm** | |
| 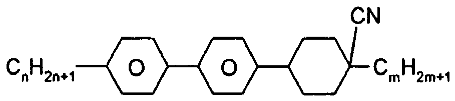 **NCB-nm** | |
| 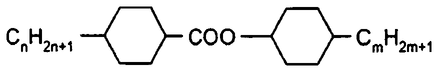 **OS-nm** | |
| 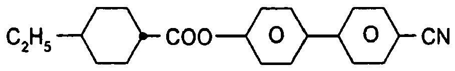 **CHE** | |
| 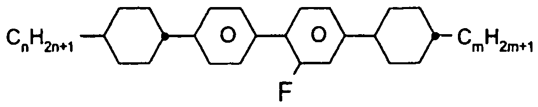 **CBC-nmF** | |
| 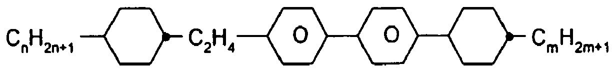 **ECBC-nm** | |
| 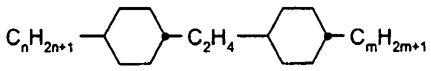 **ECCH-nm** | 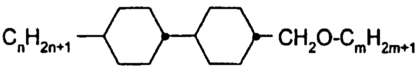 **CCH-n1EM** |
| 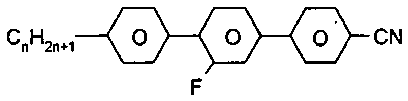 **T-nFN** | 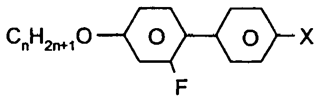 **GP-nO-X** (X = F, Cl, -OCF3 = "OT") |
| 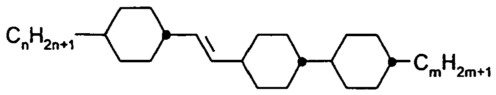 **CVCC-n-m** | |
| 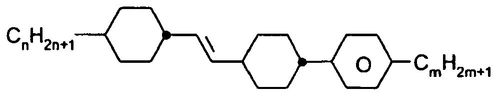 **CVCP-n-m** | |
| 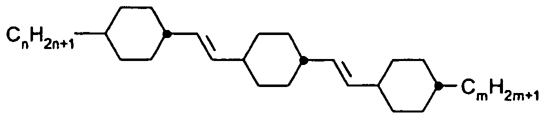 **CVCVC-n-m** | |
| 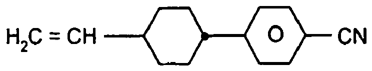 **CP-V-N** | |
| 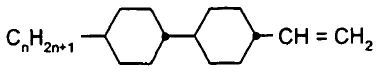 **CC-n-V** | |
| 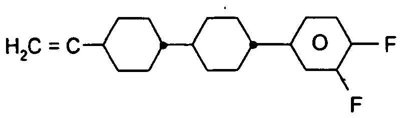 **CCG-V-F** | |
| 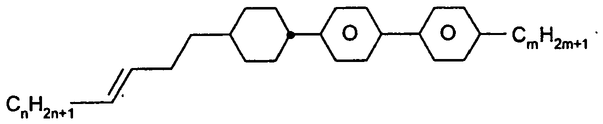 **CPP-nV2-m** | |
| 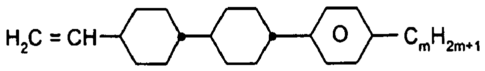 **CCP-V-m** | |
| 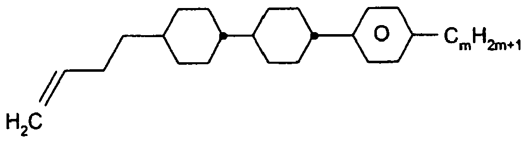 **CCP-V2-m** | |
| 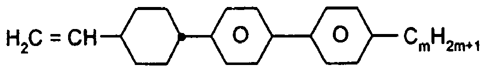 **CPP-V-m** | |
| 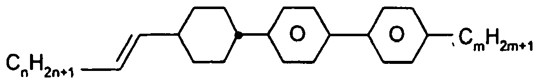 **CPP-nV-m** | |
| 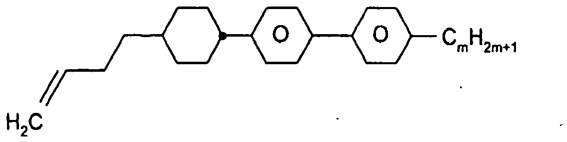 **CPP-V2-m** | |
| 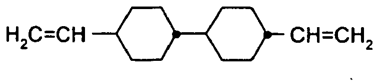 **CC-V-V** | |
| 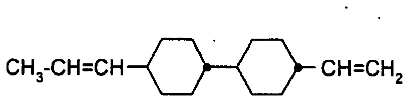 **CC-1V-V** | |
| 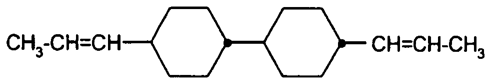 **CC-1V-V1** | |
| 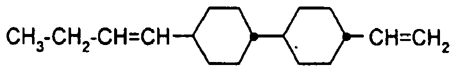 **CC-2V-V** | |
| 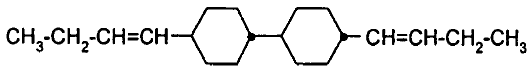 **CC-2V-V2** | |
| 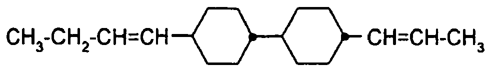 **CC-2V-V1** | |
| 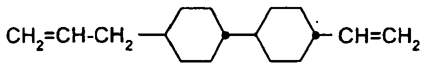 **CC-V1-V** | |
| 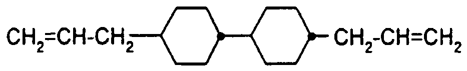 **CC-V1-1V** | |
| 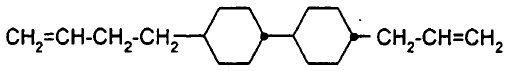 **CC-V2-1 V** | |
| 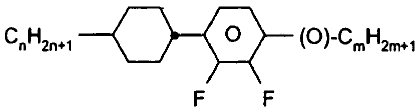 **PCH-n(O)mFF** | |
| 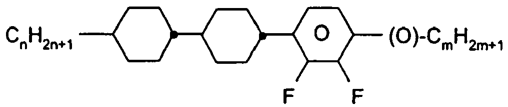 **CCP-n(O)mFF** | |
| 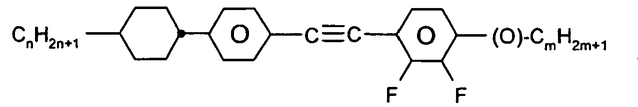 **CPTP-n(O)mFF** | |
| 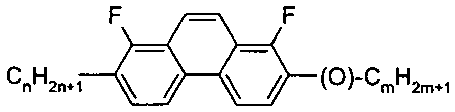 **Ph-n-(0)mFF** | |
| 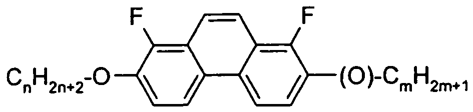 **Ph-n0-(0)mFF** | |
| 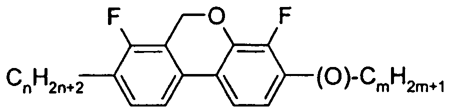 **BHHO-n-(0)mFF** | |
| 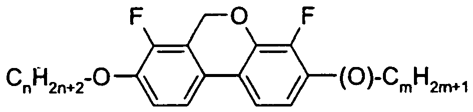 **BHHO-n0-(0)mFF** | |
| 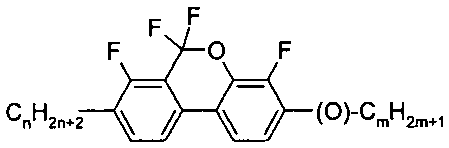 **BFFO-n-(0)mFF** | |
| 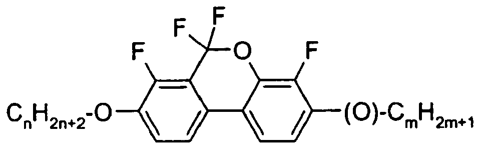 **BFFO-n0-(0)mFF** | |
| 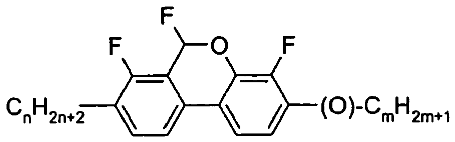 **BFO-n-(0)mFF** | |
| 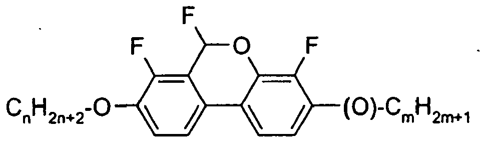 **BFO-n0-(0)mFF** | |
| 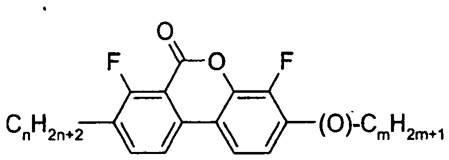 **BCOO-n-(0)mFF** | |
| 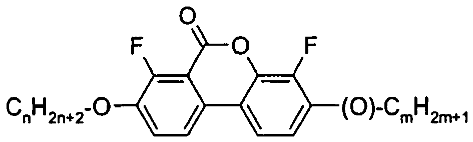 **BCOO-n0-(0)mFF** | |
| 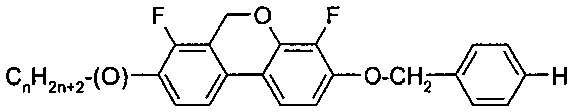 **BHHO-O1P-n(O)-HFF** | |
| 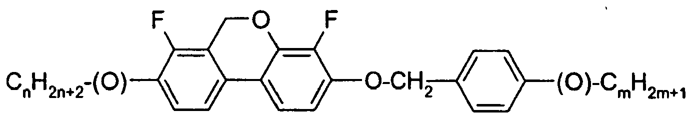 **BHHO-O1P-n(O)-(O)mFF** | |
| 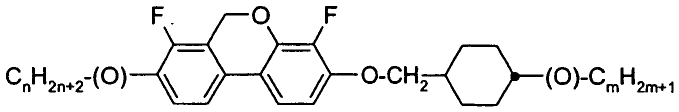 **BHHO-O1C-n(O)-(O)mFF** | |

### Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Δn bedeutet optische Anisotropie (589 nm, 20 °C), Δε die dielektrische Anisotropie (1 kHz, 20 °C), H.R. die "voltage holding ratio" (bei 100 °C, nach 5 Minuten im Ofen, 1 V), V₁₀, V₅₀ und V₉₀ (die Schwellenspannung, Mittgrauspannung bzw. Sättigungsspannung), sowie V₀ (die kapazitive Schwellenspannung) wurden jeweils bei 20 °C bestimmt.

### Substanzbeispiele

### Beispiel 1: (3-Ethoxy-6,6-difluor 8-propyl-6a,7,8,9,10,10a-hexahydro-6H-benzo[c]chromen)

### 1.1. Herstellung von 3-Ethoxy-8-propyl-7,8,9,10-tetrahydro-benzo[c]chromen-6-on

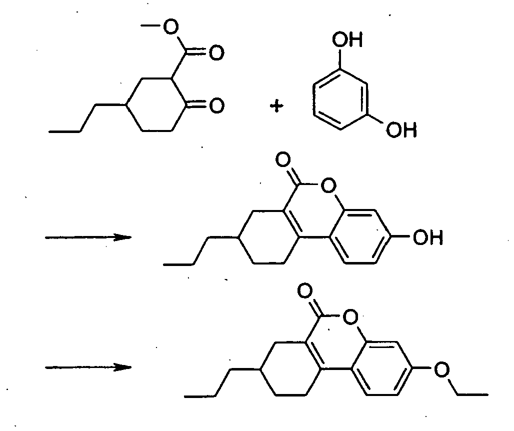

16,6 g (78,5 mmol) 2-Oxo-5-propylcyclohexancarbonsäuremethylester, 7,65 g (69,5 mmol) Resorcin und 5,6 ml (6,1 mmol) Phosphorylchlorid werden in 55 ml Toluol gelöst und 3 h unter Rückfluß erhitzt. Nach Hydrolyse mit Wasser wird der ausgefallene Niederschlag abgesaugt, mit Toluol gewaschen und getrocknet. Das Produkt wird in 200 ml Aceton gelöst, 20 g (145 mmol) Kaliumcarbonat und 9,00 g (57,7 mmol) Ethyliodid hinzugegeben und 5 h unter Rückfluß erhitzt. Das Lösungsmittel wird im Vakuum größtenteils entfernt und der Rückstand in MTB-Ether/Wasser aufgenommen. Die wäßrige Phase wird abgetrennt und mit MTB-Ether extrahiert. Die vereinigten organischen Phasen werden mit Wasser gewaschen und über Natriumsulfat getrocknet. Das Lösungsmittel wird im Vakuum entfernt und das Rohprodukt aus Ethanol umkristallisiert. Man erhält 3-Ethoxy-8-propyl-7,8,9,10-tetrahydro-benzo[c]chromen-6-on als farblose Kristalle. Der Schmelzpunkt beträgt 108°C. Die weiteren physikalischen Eigenschaften sind:
Δε = -7,5 (extrapoliert aus 5 %-iger Lösung in ZLI-2857), Δn = 0,1302 5 % in ZLI-4792),

### 1.2. Herstellung von 3-Ethoxy-8-propyl-6a,7,8,9,10,10a-hexahydro-benzo[c]chromen-6-on

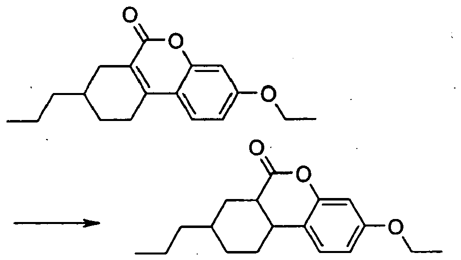

8,60 g (30 mmol) 3-Ethoxy-8-propyl-7,8,9,10-tetrahydro-benzo[c]chromen-6-on werden in THF gelöst und an Palldium-Aktivkohle bis zum Stillstand hydriert. Die Lösung wird filtriert und eingeengt. Man erhält 3-Ethoxy-8-propyl-6a,7,8,9,10,10a-hexahydro- benzo[c]chromen-6-on als farbloses Öl.

### 1.3. Herstellung von 10-Ethoxy-7-oxa-17-propyl-1,5-dithia-14,15,16,17,18,19-hexahydro-dibenzospiro[5.5]nonadecan

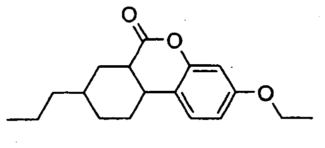

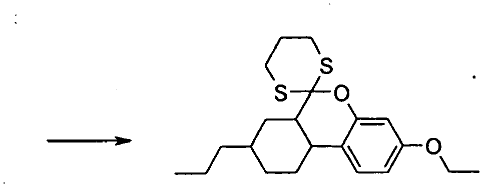

29 ml (58 mmol) einer 2 M Lösung von Trimethylaluminium in Heptan werden unter Stickstoff in 35 ml Dichlormethan vorgelegt, auf-75°C gekühlt und dann wird eine Lösung von 2,9 ml (28,5 mmol) 1,3-Propandithiol in 15 ml Dichlormethan zugetropft. Der Ansatz wird auftauen gelassen, auf -20 °C gekühlt und eine Lösung von 8,20 g (26,0 mmol) 3-Ethoxy-8-propyl-6a,7,8,9,10,10a-hexahydrobenzo[c]chromen-6-on in 10 ml Dichlormethan zugetropft. Der Ansatz wird über Nacht bei Raumtemp. rühren gelassen, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand mit Heptan/MTB (8:2) über Kieselgel filtriert. Man erhält 10,9 g Dithioorthoester als gelbes Öl, das ohne weitere Aufreinigung in der nächsten Stufe eingesetzt wird.

### 1.4. Herstellung von 3-Ethoxy-6,6-difluor-6a,7,8,9,10,10a-hexahydro-8-propyl-6H-benzo[c]chromen

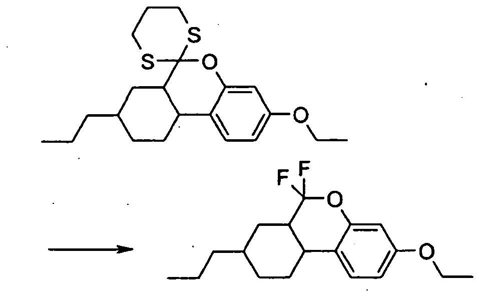

4,14 g (10,9 mmol) Dithioorthoester werden bei -70°C in 300 ml Dichlormethan vorgelegt und 8,9 ml (55 mmol) Triethylamin-trishydrofluorid hinzugegeben. Anschließend wird eine Suspension von 15,7 g (55 mmol) Dibromdimethylhydantoin in 200 ml Dichlormethan portionsweise hinzugegeben und der Ansatz wird 2 h rühren gelassen. Dann wird die Kühlung entfernt und die Lösung auf eine eiskalte Mischung aus 400 ml 1 M Natronlauge und 20 ml 39 %-iger Natriumhydrogensulfitlsg. gegeben. Die wäßrige Phase wird abgetrennt und dreimal mit Dichlormethan extrahiert. Die vereinigten organischen Phasen werden mit Wasser gewaschen und über Natriumsulfat getrocknet. Nach Entfernung des Lösungmittels im Vakuum erhält man ein gelbes Öl. Dieses wird in THF aufgenommen und an Palladium-Aktivkohlekatalysator bis zum Stillstand hydriert. Nach Filtration wird die erhaltene Lösung im Vakuum eingeengt und der Rückstand chromatographisch gereinigt. Man erhält 3-Ethoxy-6,6-difluor-6a,7,8,9,10,10a-hexahydro-8-propyl-6H-benzo[c]chromen als farbloses Öl mit den folgenden Eigenschaften.
T_{g} = -39°C.
**¹⁹F-NMR (235 MHz, CDCl₃)**
δ= -66,8 ppm (d, ²*J* = 155 Hz, 1 F, CF₂O), -81,8 (d, ²*J* = 155 Hz, 1 F, CF₂O).
**MS (EI)**

m/z (%) = 310 (98) [M⁺], 225 (100).

### Beispiel 2: (3-Ethoxy-4,6,6-trifluor-8-propyl-6a,7,8,9,10,10a-hexahydro-6H-benzo[c]chromen)

### 2.1. Herstellung von 3-Ethoxy-4-fluor-8-propyl-7,8,9,10-tetrahydro-benzo[c]chromen-6-on

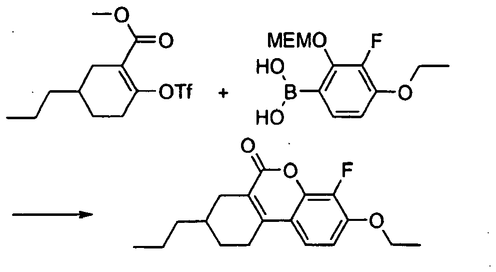

18,2 g (56,6 mmol) 5-Propyl-2-trifluormethansulfonyloxycyclohex-1-encarbonsäuremethylester, 21,5 g (74,6 mmol) 4-Ethoxy-3-fluor-2-(2-methoxy-ethoxymethoxy)-benzolboronsäure, 1,5 ml Wasser, 33 g (120 mmol) Natriummetaborat, 1,12 g (1,6 mmol) Bis(triphenylphosphin)palladium(II)chlorid und 0,1 ml (1,6 mmol) Hydraziniumhydroxid werden in 300 ml Tetrahydrofuran über Nacht unter Rückfluß erhitzt. Nach Zugabe von Wasser wird die wäßrige Phase abgetrennt und zweimal mit MTB-Ether extrahiert. Die vereinigten organischen Phasen werden mit gesättigter Natriumchloridlsg. gewaschen und über Natriumsulfat getrocknet. Das Lösungsmittel wird im Vakuum entfernt und der Rückstand mit n-Heptan/MTB-Ether (6:4) über Kieselgel filtriert. Man erhält 3-Ethoxy-4-fluor-8-propyl-7,8,9,10-tetrahydro-benzo[c]chromen-6-on als farblose Kristalle.
**¹³C-NMR (CDCl₃, 75 MHz)**
δ = 14,2 ppm (CH₃), 14,8 (CH₃), 19,9 (CH₂), 25,4 (CH₂), 27,5 (CH₂), 30,4 (CH₂), 32,6 (CH), 32,7 (CH₂), 38,3 (CH₂), 65,6 (OCH₂CH₃), 109,8 (CH), 114,870 (C), 117,7 (d, *J* = 4,4 Hz, CH), 121,184 (C), 139,7 (d, *J* = 250 Hz, CF), 146,860 (C), 148,384 (C), 148,485 (C), 160,755, (C=O).

### 2.2. Herstellung von 3-Ethoxy-4,6,6-trifluor-8-propyl-6a,7,8,9,10,10a-hexahydro-6H-benzo[c]chromen

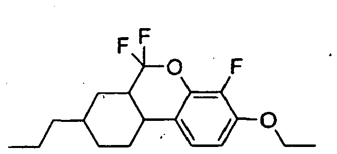

In Analogie zu der unter 1. beschriebenen Synthese erhält man 3-Ethoxy-4,6,6-trifluor-8-propyl-6a,7,8,9,10,10a-hexahydro-6H-benzo[c]chromen.

### Referenz-Beispiele 3 bis 120

Analog zu Beispiel 1.2. werden hergestellt:

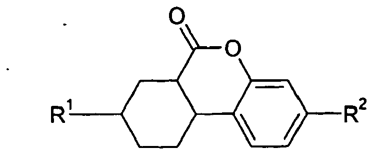

Bemerkung: * 10%-iger Lösung in ZLI-4792, bzw. ZLI-2857 (Δε) extrapolierte Werte.

| Nr. | R¹ | R² | Phasensequenz Δε* T/°C | T*(N,I) /°C |
|---|---|---|---|---|
| 3 | CH₃ | CH₃ | | |
| 4 | CH₃ | C₂H₅ | | |
| 5 | CH₃ | *n*-C₃H₇ | | |
| 6 | CH₃ | *n*-C₄H₉ | | |
| 7 | CH₃ | *n*-C₅H₁₁ | | |
| 8 | CH₃ | *n*-C₆H₁₃ | | |
| 9 | CH₃ | *n*-C₇H₁₅ | | |
| 10 | CH₃ | CH₃O | | |
| 11 | CH₃ | C₂H₅O | | |
| 12 | CH₃ | *n*-C₃H₇O | | |
| 13 | CH₃ | *n*-C₄H₉O | | |
| 14 | CH₃ | CH₂=CH | | |
| 15 | CH₃ | *E*-CH₃-CH=CH | | |
| 16 | CH₃ | CH₂=CH-O | | |
| 17 | CH₃ | CH₂=CH-CH₂O | | |
| 18 | C₂H₅ | CH₃ | | |
| 19 | C₂H₅ | C₂H₅ | | |
| 20 | C₂H₅ | *n*-C₃H₇ | | |
| 21 | C₂H₅ | *n*-C₄H₉ | | |
| 22 | C₂H₅ | *n*-C₅H₁₁ | | |
| 23 | C₂H₅ | *n*-C₆H₁₃ | | |
| 24 | C₂H₅ | *n*-C₇H₁₅ | | |
| 25 | C₂H₅ | CH₃O | | |
| 26 | C₂H₅ | C₂H₅O | | |
| 27 | C₂H₅ | *n*-C₃H₇O | | |
| 28 | C₂H₅ | *n*-C₄H₉O | | |
| 29 | C₂H₅ | CH₂=CH | | |
| 30 | C₂H₅ | *E*-CH₃-CH=CH | | |
| 31 | C₂H₅ | CH₂=CH-O | | |
| 32 | C₂H₅ | CH₂=CH-CH₂O | | |
| 33 | *n*-C₃H₇ | CH₃ | | |
| 34 | *n*-C₃H₇ | C₂H₅ | | |
| 35 | *n*-C₃H₇ | *n*-C₃H₇ | | |
| 36 | *n*-C₃H₇ | *n*-C₄H₉ | | |
| 37 | *n*-C₃H₇ | *n*-C₅H₁₁ | | |
| 38 | *n*-C₃H₇ | *n*-C₆H₁₃ | | |
| 39 | *n*-C₃H₇ | *n*-C₇H₁₅ | | |
| 40 | *n*-C₃H₇ | CH₃O | | |
| 1.2 | *n*-C₃H₇ | C₂H₅O | | |
| 41 | *n*-C₃H₇ | *n*-C₃H₇O | | |
| 42 | *n*-C₃H₇ | *n*-C₄H₉O | | |
| 43 | *n*-C₃H₇ | CH₂=CH | | |
| 44 | *n*-C₃H₇ | *E*-CH₃-CH=CH | | |
| 45 | *n*-C₃H₇ | CH₂=CH-O | | |
| 46 | *n*-C₃H₇ | CH₂=CH-CH₂O | | |
| 47 | *n*-C₄H₉ | CH₃ | | |
| 48 | *n*-C₄H₉ | C₂H₅ | | |
| 49 | *n*-C₄H₉ | *n*-C₃H₇ | | |
| 50 | *n*-C₄H₉ | *n*-C₄H₉ | | |
| 51 | *n*-C₄H₉ | *n*-C₅H₁₁ | | |
| 52 | *n*-C₄H₉ | *n*-C₆H₁₃ | | |
| 53 | *n*-C₄H₉ | *n*-C₇H₁₅ | | |
| 54 | *n*-C₄H₉ | CH₃O | | |
| 55 | *n*-C₄H₉ | C₂H₅O | | |
| 56 | *n*-C₄H₉ | *n*-C₃H₇O | | |
| 57 | *n*-C₄H₉ | *n*-C₄H₉O | | |
| 58 | *n*-C₄H₉ | CH₂=CH | | |
| 59 | *n*-C₄H₉ | *E*-CH₃-CH=CH | | |
| 60 | *n*-C₄H₉ | CH₂=CH-O | | |
| 61 | *n*-C₄H₉ | CH₂=CH-CH₂O | | |
| 62 | CH₃O | CH₃ | | |
| 63 | CH₃O | C₂H₅ | | |
| 64 | CH₃O | *n*-C₃H₇ | | |
| 65 | CH₃O | *n*-C₄H₉ | | |
| 66 | CH₃O | *n*-C₅H₁₁ | | |
| 67 | CH₃O | *n*-C₆H₁₃ | | |
| 68 | CH₃O | *n*-C₇H₁₅ | | |
| 69 | CH₃O | CH₃O | | |
| 70 | CH₃O | C₂H₅O | | |
| 71 | CH₃O | *n*-C₃H₇O | | |
| 72 | CH₃O | *n*-C₄H₉O | | |
| 73 | CH₃O | CH₂=CH | | |
| 74 | CH₃O | *E*-CH₃-CH=CH | | |
| 75 | CH₃O | CH₂=CH-O | | |
| 76 | CH₃O | CH₂=CH-CH₂O | | |
| 77 | C₂H₅O | CH₃ | | |
| 78 | C₂H₅O | C₂H₅ | | |
| 79 | C₂H₅O | *n*-C₃H₇ | | |
| 80 | C₂H₅O | *n*-C₄H₉ | | |
| 81 | C₂H₅O | *n*-C₅H₁₁ | C 137 I | |
| 82 | C₂H₅O | *n*-C₆H₁₃ | | |
| 83 | C₂H₅O | *n*-C₇H₁₅ | | |
| 84 | C₂H₅O | CH₃O | | |
| 85 | C₂H₅O | C₂H₅O | | |
| 86 | C₂H₅O | *n*-C₃H₇O | | |
| 87 | C₂H₅O | *n*-C₄H₉O | | |
| 88 | C₂H₅O | CH₂=CH | | |
| 89 | C₂H₅O | *E*-CH₃-CH=CH | | |
| 90 | C₂H₅O | CH₂=CH-O | | |
| 91 | C₂H₅O | CH₂=CH-CH₂O | | |
| 92 | CH₂=CH | CH₃ | | |
| 93 | CH₂₌CH | C₂H₅ | | |
| 94 | CH₂=CH | *n*-C₃H₇ | | |
| 95 | CH₂=CH | *n*-C₄H₉ | | |
| 96 | CH₂=CH | *n*-C₅H₁₁ | | |
| 97 | CH₂=CH | *n*-C₆H₁₃ | | |
| 98 | CH₂=CH | *n*-C₇H₁₅ | | |
| 99 | CH₂=CH | CH₃O | | |
| 100 | CH₂=CH | C₂H₅O | | |
| 101 | CH₂=CH | *n*-C₃H₇O | | |
| 102 | CH₂=CH | *n*-C₄H₉O | | |
| 103 | CH₂=CH | CH₂=CH | | |
| 104 | CH₂=CH | *E*-CH₃-CH=CH | | |
| 105 | CH₂=CH | CH₂=CH-O | | |
| 106 | CH₂=CH | CH₂=CH-CH₂O | | |
| 107 | CH₂=CH-O | CH₃ | | |
| 108 | CH₂=CH-O | C₂H₅ | | |
| 109 | CH₂=CH-O | *n*-C₃H₇ | | |
| 110 | CH₂=CH-O | *n*-C₄H₉ | | |
| 111 | CH₂=CH-O | *n*-C₅H₁₁ | | |
| 112 | CH₂=CH-O | *n*-C₆H₁₃ | | |
| 113 | CH₂=CH-O | *n*-C₇H₁₅ | | |
| 114 | CH₂=CH-O | CH₃O | | |
| 115 | CH₂=CH-O | C₂H₅O | | |
| 116 | CH₂=CH-O | *n*-C₃H₇O | | |
| 117 | CH₂=CH-O | *n*-C₄H₉O | | |
| 118 | CH₂=CH-O | CH₂=CH | | |
| 119 | CH₂=CH-O | *E*-CH₃-CH=CH | | |
| 120 | CH₂=CH-O | CH₂=CH-O | | |
| 121 | CH₂=CH-O | CH₂=CH-CH₂O | | |

### Beispiele 122 bis 240

Analog zu Beispiel 1.4. werden hergestellt: 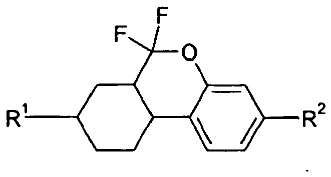 Bemerkung: * 10%-iger Lösung in ZLI-4792, bzw. ZLI-2857 (Δε) extrapolierte Werte.

| Nr. | R¹ | R² | Phasensequenz T/°C | Δε* | T*(N,I) /°C |
|---|---|---|---|---|---|
| 122 | CH₃ | CH₃ | | | |
| 123 | CH₃ | C₂H₅ | | | |
| 124 | CH₃ | *n*-C₃H₇ | | | |
| 125 | CH₃ | *n*-C₄H₉ | | | |
| 121 | CH₃ | *n*-C₅H₁₁ | | | |
| 126 | CH₃ | *n*-C₆H₁₃ | | | |
| 127 | CH₃ | *n*-C₇H₁₅ | | | |
| 128 | CH₃ | CH₃O | | | |
| 129 | CH₃ | C₂H₅O | | | |
| 130 | CH₃ | *n*-C₃H₇O | | | |
| 131 | CH₃ | *n*-C₄H₉O | | | |
| 132 | CH₃ | CH₂=CH | | | |
| 133 | CH₃ | *E*-CH₃-CH=CH | | | |
| 134 | CH₃ | CH₂=CH-O | | | |
| 135 | CH₃ | CH₂=CH-CH₂O | | | |
| 136 | C₂H₅ | CH₃ | | | |
| 137 | C₂H₅ | C₂H₅ | | | |
| 138 | C₂H₅ | *n*-C₃H₇ | | | |
| 139 | C₂H₅ | *n*-C₄H₉ | | | |
| 140 | C₂H₅ | *n*-C₅H₁₁ | | | |
| 141 | C₂H₅ | *n*-C₆H₁₃ | | | |
| 142 | C₂H₅ | *n*-C₇H₁₅ | | | |
| 143 | C₂H₅ | CH₃O | | | |
| 144 | C₂H₅ | C₂H₅O | | | |
| 145 | C₂H₅ | *n*-C₃H₇O | | | |
| 146 | C₂H₅ | *n*-C₄H₉O | | | |
| 147 | C₂H₅ | CH₂=CH | | | |
| 148 | C₂H₅ | *E*-CH₃-CH=CH | | | |
| 149 | C₂H₅ | CH₂=CH-O | | | |
| 150 | C₂H₅ | CH₂=CH-CH₂O | | | |
| 151 | *n*-C₃H₇ | CH₃ | | | |
| 152 | *n*-C₃H₇ | C₂H₅ | | | |
| 153 | *n*-C₃H₇ | *n*-C₃H₇ | | | |
| 154 | *n*-C₃H₇ | *n*-C₄H₉ | | | |
| 155 | *n*-C₃H₇ | *n*-C₅H₁₁ | | | |
| 156 | *n*-C₃H₇ | *n*-C₆H₁₃ | | | |
| 157 | *n*-C₃H₇ | *n*-C₇H₁₅ | | | |
| 158 | *n*-C₃H₇ | CH₃O | | | |
| 159 | *n*-C₃H₇ | C₂H₅O | | | |
| 1.4 | *n*-C₃H₇ | *n*-C₃H₇O | T_{g} = -39°C | | |
| 160 | *n*-C₃H₇ | *n*-C₄H₉O | | | |
| 161 | *n*-C₃H₇ | CH₂=CH | | | |
| 162 | *n*-C₃H₇ | *E*-CH₃-CH=CH | | | |
| 163 | *n*-C₃H₇ | CH₂=CH-O | | | |
| 164 | *n*-C₃H₇ | CH₂=CH-CH₂O | | | |
| 165 | *n*-C₄H₉ | CH₃ | | | |
| 166 | *n*-C₄H₉ | C₂H₅ | | | |
| 167 | *n*-C₄H₉ | *n*-C₃H₇ | | | |
| 168 | *n*-C₄H₉ | *n*-C₄H₉ | | | |
| 169 | *n*-C₄H₉ | *n*-C₅H₁₁ | | | |
| 170 | *n*-C₄H₉ | *n*-C₆H₁₃ | | | |
| 171 | *n*-C₄H₉ | *n*-C₇H₁₅ | | | |
| 172 | *n*-C₄H₉ | CH₃O | | | |
| 173 | *n*-C₄H₉ | C₂H₅O | | | |
| 174 | *n*-C₄H₉ | *n*-C₃H₇O | | | |
| 175 | *n*-C₄H₉ | *n*-C₄H₉O | | | |
| 176 | *n*-C₄H₉ | CH₂=CH | | | |
| 177 | *n*-C₄H₉ | *E*-CH₃-CH=CH | | | |
| 178 | *n*-C₄H₉ | CH₂=CH-O | | | |
| 179 | *n*-C₄H₉ | CH₂=CH-CH₂O | | | |
| 180 | CH₃O | CH₃ | | | |
| 181 | CH₃O | C₂H₅ | | | |
| 182 | CH₃O | *n*-C₃H₇ | | | |
| 183 | CH₃O | *n*-C₄H₉ | | | |
| 184 | CH₃O | *n*-C₅H₁₁ | | | |
| 185 | CH₃O | *n*-C₆H₁₃ | | | |
| 186 | CH₃O | *n*-C₇H₁₅ | | | |
| 187 | CH₃O | CH₃O | | | |
| 188 | CH₃O | C₂H₅O | | | |
| 189 | CH₃O | *n*-C₃H₇O | | | |
| 190 | CH₃O | *n*-C₄H₉O | | | |
| 191 | CH₃O | CH₂=CH | | | |
| 192 | CH₃O | *E*-CH₃-CH=CH | | | |
| 193 | CH₃O | CH₂=CH-O | | | |
| 194 | CH₃O | CH₂=CH-CH₂O | | | |
| 195 | C₂H₅O | CH₃ | | | |
| 196 | C₂H₅O | C₂H₅ | | | |
| 197 | C₂H₅O | *n*-C₃H₇ | | | |
| 198 | C₂H₅O | *n*-C₄H₉ | | | |
| 199 | C₂H₅O | *n*-C₅H₁₁ | | | |
| 201 | C₂H₅O | *n*-C₆H₁₃ | | | |
| 202 | C₂H₅O | *n*-C₇M₁₅ | | | |
| 203 | C₂H₅O | CH₃O | | | |
| 204 | C₂H₅O | C₂H₅O | | | |
| 205 | C₂H₅O | *n*-C₃H₇O | | | |
| 206 | C₂H₅O | *n*-C₄H₉O | | | |
| 207 | C₂H₅O | CH₂=CH | | | |
| 208 | C₂H₅O | *E*-CH₃-CH=CH | | | |
| 209 | C₂H₅O | CH₂=CH-O | | | |
| 210 | C₂H₅O | CH₂=CH-CH₂O | | | |
| 211 | CH₂=CH | CH₃ | | | |
| 212 | CH₂=CH | C₂H₅ | | | |
| 213 | CH₂=CH | *n*-C₃H₇ | | | |
| 214 | CH₂=CH | *n*-C₄H₉ | | | |
| 215 | CH₂=CH | *n*-C₅H₁₁ | | | |
| 216 | CH₂=CH | *n*-C₅H₁₃ | | | |
| 217 | CH₂=CH | *n*-C₇H₁₅ | | | |
| 218 | CH₂=CH | CH₃O | | | |
| 219 | CH₂=CH | C₂H₅O | | | |
| 220 | CH₂=CH | *n*-C₃H₇O | | | |
| 221 | CH₂=CH | *n*-C₄H₉O | | | |
| 222 | CH₂=CH | CH₂=CH | | | |
| 223 | CH₂=CH | *E*-CH₃-CH=CH | | | |
| 224 | CH₂=CH | CH₂=CH-O | | | |
| 225 | CH₂=CH | CH₂=CH-CH₂O | | | |
| 226 | CH₂=CH-O | CH₃ | | | |
| 227 | CH₂=CH-O | C₂H₅ | | | |
| 228 | CH₂=CH-O | *n*-C₃H₇ | | | |
| 229 | CH₂=CH-O | *n*-C₄H₉ | | | |
| 230 | CH₂=CH-O | *n*-C₅H₁₁ | | | |
| 231 | CH₂=CH-O | *n*-C₅H₁₃ | | | |
| 232 | CH₂=CH-O | *n*-C₇H₁₅ | | | |
| 233 | CH₂=CH-O | CH₃O | | | |
| 234 | CH₂=CH-O | C₂H₅O | | | |
| 235 | CH₂=CH-O | *n*-C₃H₇O | | | |
| 236 | CH₂=CH-O | *n*-C₄H₉O | | | |
| 237 | CH₂=CH-O | CH₂=CH | | | |
| 238 | CH₂=CH-O | *E*-CH₃-CH=CH | | | |
| 239 | CH₂=CH-O | CH₂=CH-O | | | |
| 240 | CH₂=CH-O | CH₂=CH-CH₂O | | | |

### Referenz-Beispiele 241 bis 359

Analog zu Beispiel 1.2. werden hergestellt: 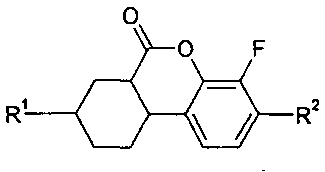 Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 241 | CH₃ | CH₃ | | |
| 242 | CH₃ | C₂H₅ | | |
| 243 | CH₃ | *n*-C₃H₇ | | |
| 244 | CH₃ | *n*-C₄H₉ | | |
| 245 | CH₃ | *n*-C₅H₁₁ | | |
| 246 | CH₃ | *n*-C₆H₁₃ | | |
| 247 | CH₃ | *n*-C₇H₁₅ | | |
| 248 | CH₃ | CH₃O | | |
| 249 | CH₃ | C₂H₅O | | |
| 250 | CH₃ | *n*-C₃H₇O | | |
| 251 | CH₃ | *n*-C₄H₉O | | |
| 252 | CH₃ | CH₂=CH | | |
| 253 | CH₃ | *E*-CH₃-CH=CH | | |
| 254 | CH₃ | CH₂=CH-O | | |
| 255 | CH₃ | CH₂=CH-CH₂O | | |
| 256 | C₂H₅ | CH₃ | | |
| 257 | C₂H₅ | C₂H₅ | | |
| 258 | C₂H₅ | *n*-C₃H₇ | | |
| 259 | C₂H₅ | *n*-C₄H₉ | | |
| 260 | C₂H₅ | *n*-C₅H₁₁ | | |
| 261 | C₂H₅ | *n*-C₆H₁₃ | | |
| 262 | C₂H₅ | *n*-C₇H₁₅ | | |
| 263 | C₂H₅ | CH₃O | | |
| 264 | C₂H₅ | C₂H₅O | | |
| 265 | C₂H₅ | *n*-C₃H₇O | | |
| 266 | C₂H₅ | *n*-C₄H₉O | | |
| 267 | C₂H₅ | CH₂=CH | | |
| 268 | C₂H₅ | *E*-CH₃-CH=CH | | |
| 269 | C₂H₅ | CH₂=CH-O | | |
| 270 | C₂H₅ | CH₂=CH-CH₂O | | |
| 271 | *n*-C₃H₇ | CH₃ | | |
| 272 | *n*-C₃H₇ | C₂H₅ | | |
| 273 | *n*-C₃H₇ | *n*-C₃H₇ | | |
| 274 | *n*-C₃H₇ | *n*-C₄H₉ | | |
| 275 | *n*-C₃H₇ | *n*-C₅H₁₁ | | |
| 276 | *n*-C₃H₇ | *n*-C₆H₁₃ | | |
| 277 | *n*-C₃H₇ | *n*-C₇H₁₅ | | |
| 278 | *n*-C₃H₇ | CH₃O | | |
| 279 | *n*-C₃H₇ | C₂H₅O | | |
| 280 | *n*-C₃H₇ | *n*-C₃H₇O | | |
| 281 | *n*-C₃H₇ | *n*-C₄H₉O | | |
| 282 | *n*-C₃H₇ | CH₂=CH | | |
| 283 | *n*-C₃H₇ | *E*-CH₃-CH=CH | | |
| 284 | *n*-C₃H₇ | CH₂=CH-O | | |
| 285 | *n*-C₃H₇ | CH₂=CH-CH₂O | | |
| 286 | *n*-C₄H₉ | CH₃ | | |
| 287 | *n*-C₄H₉ | C₂H₅ | | |
| 288 | *n*-C₄H₉ | *n*-C₃H₇ | | |
| 289 | *n*-C₄H₉ | *n*-C₄H₉ | | |
| 290 | *n*-C₄H₉ | *n*-C₅H₁₁ | | |
| 291 | *n*-C₄H₉ | *n*-C₆H₁₃ | | |
| 292 | *n*-C₄H₉ | *n*-C₇H₁₅ | | |
| 293 | *n*-C₄H₉ | CH₃O | | |
| 294 | *n*-C₄H₉ | C₂H₅O | | |
| 295 | *n*-C₄H₉ | *n*-C₃H₇O | | |
| 296 | *n*-C₄H₉ | *n*-C₄H₉O | | |
| 297 | *n*-C₄H₉ | CH₂=CH | | |
| 298 | *n*-C₄H₉ | *E*-CH₃-CH=CH | | |
| 299 | *n*-C₄H₉ | CH₂=CH-O | | |
| 300 | *n*-C₄H₉ | CH₂=CH-CH₂O | | |
| 300 | CH₃O | CH₃ | | |
| 302 | CH₃O | C₂H₅ | | |
| 303 | CH₃O | *n*-C₃H₇ | | |
| 304 | CH₃O | *n*-C₄H₉ | | |
| 305 | CH₃O | *n*-C₅H₁₁ | | |
| 306 | CH₃O | *n*-C₆H₁₃ | | |
| 307 | CH₃O | *n*-C₇H₁₅ | | |
| 308 | CH₃O | CH₃O | | |
| 309 | CH₃O | C₂H₅O | | |
| 310 | CH₃O | *n*-C₃H₇O | | |
| 311 | CH₃O | *n*-C₄H₉O | | |
| 312 | CH₃O | CH₂=CH | | |
| 313 | CH₃O | *E*-CH₃-CH=CH | | |
| 314 | CH₃O | CH₂=CH-O | | |
| 315 | CH₃O | CH₂=CH-CH₂O | | |
| 316 | C₂H₅O | CH₃ | | |
| 317 | C₂H₅O | C₂H₅ | | |
| 318 | C₂H₅O | *n*-C₃H₇ | | |
| 319 | C₂H₅O | *n*-C₄H₉ | | |
| 320 | C₂H₅O | *n*-C₅H₁₁ | | |
| 241 | C₂H₅O | *n*-C₆H₁₃ | | |
| 321 | C₂H₅O | *n*-C₇H₁₅ | | |
| 322 | C₂H₅O | CH₃O | | |
| 323 | C₂H₅O | C₂H₅O | | |
| 324 | C₂H₅O | *n*-C₃H₇O | | |
| 325 | C₂H₅O | *n*-C₄H₉O | | |
| 326 | C₂H₅O | CH₂=CH | | |
| 327 | C₂H₅O | *E*-CH₃-CH=CH | | |
| 328 | C₂H₅O | CH₂=CH-O | | |
| 329 | C₂H₅O | CH₂=CH-CH₂O | | |
| 330 | CH₂=CH | CH₃ | | |
| 331 | CH₂=CH | C₂H₅ | | |
| 332 | CH₂=CH | *n*-C₃H₇ | | |
| 333 | CH₂=CH | *n*-C₄H₉ | | |
| 334 | CH₂=CH | *n*-C₅H₁₁ | | |
| 335 | CH₂=CH | *n*-C₆H₁₃ | | |
| 336 | CH₂=CH | *n*-C₇H₁₅ | | |
| 337 | CH₂=CH | CH₃O | | |
| 338 | CH₂=CH | C₂H₅O | | |
| 339 | CH₂=CH | *n*-C₃H₇O | | |
| 340 | CH₂=CH | *n*-C₄H₉O | | |
| 341 | CH₂=CH | CH₂=CH | | |
| 342 | CH₂=CH | *E*-CH₃-CH=CH | | |
| 343 | CH₂=CH | CH₂=CH-O | | |
| 344 | CH₂=CH | CH₂=CH-CH₂O | | |
| 345 | CH2=CH-0 | CH₃ | | |
| 346 | CH₂=CH-O | C₂H₅ | | |
| 347 | CH₂=CH-O | *n*-C₃H₇ | | |
| 348 | CH₂=CH-O | *n*-C₄H₉ | | |
| 349 | CH₂=CH-O | *n*-C₅H₁₁ | | |
| 350 | CH₂=CH-O | *n*-C₆H₁₃ | | |
| 351 | CH₂=CH-O | *n*-C₇H₁₅ | | |
| 352 | CH₂=CH-O | CH₃O | | |
| 353 | CH₂=CH-O | C₂H₅O | | |
| 354 | CH₂=CH-O | *n*-C₃H₇O | | |
| 355 | CH₂=CH-O | *n*-C₄H₉O | | |
| 356 | CH₂=CH-O | CH₂=CH | | |
| 357 | CH₂=CH-O | *E*-CH₃-CH=CH | | |
| 358 | CH₂=CH-O | CH₂=CH-O | | |
| 359 | CH₂=CH-O | CH₂=CH-CH₂O | | |

### Beispiele 360 bis 479

Analog zu Beispiel 2.2. werden hergestellt:

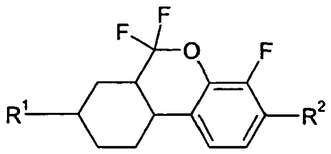

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 360 | CH₃ | CH₃ | | |
| 361 | CH₃ | C₂H₅ | | |
| 362 | CH₃ | *n*-C₃H₇ | | |
| 363 | CH₃ | *n*-C₄H₉. | | |
| 364 | CH₃ | *n*-C₅H₁₁ | | |
| 365 | CH₃ | *n*-C₆H₁₃ | | |
| 366 | CH₃ | *n*-C₇H₁₅ | | |
| 367 | CH₃ | CH₃O | | |
| 368 | CH₃ | C₂H₅O | | |
| 369 | CH₃ | *n*-C₃H₇O | | |
| 370 | CH₃ | *n*-C₄H₉O | | |
| 371 | CH₃ | CH₂=CH | | |
| 372 | CH₃ | *E*-CH₃-CH=CH | | |
| 373 | CH₃ | CH₂=CH-O | | |
| 374 | CH₃ | CH₂=CH-CH₂O | | |
| 375 | C₂H₅ | CH₃ | | |
| 376 | C₂H₅ | C₂H₅ | | |
| 377 | C₂H₅ | *n*-C₃H₇ | | |
| 378 | C₂H₅ | *n*-C₄H₉ | | |
| 379 | C₂H₅ | *n*-C₅H₁₁ | | |
| 380 | C₂H₅ | *n*-C₆H₁₃ | | |
| 381 | C₂H₅ | *n*-C₇H₁₅ | | |
| 382. | C₂H₅ | CH₃O | | |
| 383 | C₂H₅ | C₂H₅O | | |
| 384 | C₂H₅ | *n*-C₃H₇O | | |
| 385 | C₂H₅ | *n*-C₄H₉O | | |
| 386 | C₂H₅ | CH₂=CH | | |
| 387 | C₂H₅ | *E*-CH₃=CH=CH | | |
| 388 | C₂H₅ | CH₂=CH-O | | |
| 389 | C₂H₅ | CH₂=CH-CH₂O | | |
| 390 | *n*-C₃H₇ | CH₃ | | |
| 391 | *n*-C₃H₇ | C₂H₅ | | |
| 392 | *n*-C₃H₇ | *n*-C₃H₇ | | |
| 393 | *n*-C₃H₇ | *n*-C₄H₉ | | |
| 394 | *n*-C₃H₇ | *n*-C₅H₁₁ | | |
| 395 | *n*-C₃H₇ | *n*-C₆H₁₃ | | |
| 396 | *n*-C₃H₇ | *n*-C₇H₁₅ | | |
| 397 | *n*-C₃H₇ | CH₃O | | |
| 398 | *n*-C₃H₇ | C₂H₅O | | -12,6 |
| 399 | *n*-C₃H₇ | *n*-C₃H₇O | | |
| 400 | *n*-C₃H₇ | *n*-C₄H₉O | | |
| 401 | *n*-C₃H₇ | CH₂=CH | | |
| 402 | *n*-C₃H₇ | *E*-CH₃-CH=CH | | |
| 403 | *n*-C₃H₇ | CH₂=CH-O | | |
| 404 | *n*-C₃H₇ | CH₂=CH-CH₂O | | |
| 405 | *n*-C₄H₉ | CH₃ | | |
| 406 | *n*-C₄H₉ | C₂H₅ | | |
| 407 | *n*-C₄H₉ | *n*-C₃H₇ | | |
| 408 | *n*-C₄H₉ | *n*-C₄H₉ | | |
| 409 | *n*-C₄H₉ | *n*-C₅H₁₁ | | |
| 410 | *n*-C₄H₉ | *n*-C₆H₁₃ | | |
| 411 | *n*-C₄H₉ | *n*-C₇H₁₅ | | |
| 412 | *n*-C₄H₉ | CH₃O | | |
| 413 | *n*-C₄H₉ | C₂H₅O | | |
| 414 | *n*-C₄H₉ | *n*-C₃H₇O | | |
| 415 | *n*-C₄H₉ | *n*-C₄H₉O | | |
| 416 | *n*-C₄H₉ | CH₂=CH | | |
| 417 | *n*-C₄H₉ | *E*-CH₃-CH=CH | | |
| 418 | *n*-C₄H₉ | CH₂=CH-O | | |
| 419 | *n*-C₄H₉ | CH₂=CH-CH₂O | | |
| 420 | CH₃O | CH₃ | | |
| 421 | CH₃O | C₂H₅ | | |
| 422 | CH₃O | *n*-C₃H₇ | | |
| 423 | CH₃O | *n*-C₄H₉ | | |
| 424 | CH₃O | *n*-C₅H₁₁ | | |
| 425 | CH₃O | *n*-C₆H₁₃ | | |
| 426 | CH₃O | *n*-C₇H₁₅ | | |
| 427 | CH₃O | CH₃O | | |
| 428 | CH₃O | C₂H₅O | | |
| 429 | CH₃O | *n*-C₃H₇O | | |
| 430 | CH₃O | *n*-C₄H₉O | | |
| 431 | CH₃O | CH₂=CH | | |
| 432 | CH₃O | *E*-CH₃-CH=CH | | |
| 453 | CH₃O | CH₂=CH-O | | |
| 434 | CH₃O | CH₂=CH-CH₂O | | |
| 435 | C₂H₅O | CH₃ | | |
| 436 | C₂H₅O | C₂H₅ | | |
| 437 | C₂HₛO | *n*-C₃H₇ | | |
| 438 | C₂H₅O | *n*-C₄H₉ | | |
| 439 | C₂H₅O | *n*-C₅H₁₁ | | |
| 440 | C₂H₅O | *n*-C₆H₁₃ | | |
| 441 | C₂H₅O | *n*-C₇H₁₅ | | |
| 442 | C₂H₅O | CH₃O | | |
| 443 | C₂H₅O | C₂H₅O | | |
| 444 | C₂H₅O | *n*-C₃H₇O | | |
| 445 | C₂H₅O | *n*-C₄H₉O | | |
| 446 | C₂H₅O | CH₂=CH | | |
| 447 | C₂H₅O | *E*-CH₃-CH=CH | | |
| 448 | C₂H₅O | CH₂=CH-O | | |
| 449 | C₂H₅O | CH₂=CH-CH₂O | | |
| 450 | CH₂=CH | CH₃ | | |
| 451 | CH₂=CH | C₂H₅ | | |
| 452 | CH₂=CH | *n*-C₃H₇ | | |
| 453 | CH₂=CH | *n*-C₄H₉ | | |
| 454 | CH₂=CH | *n*-C₅H₁₁ | | |
| 455 | CH₂=CH | *n*-C₆H₁₃ | | |
| 456 | CH₂=CH | *n*-C₇H₁₅ | | |
| 457 | CH₂=CH | CH₃O | | |
| 458 | CH₂=CH | C₂H₅O | | |
| 459 | CH₂=CH | *n*-C₃H₇O | | |
| 460 | CH₂=CH | *n*-C₄H₉O | | |
| 461 | CH₂=CH | CH₂=CH | | |
| 462 | CH₂=CH | *E*-CH₃-CH=CH | | |
| 463 | CH₂=CH | CH₂=CH-O | | |
| 464 | CH₂=CH | CH₂=CH-CH₂O | | |
| 465 | CH₂=CH-O | CH₃ | | |
| 466 | CH₂=CH-O | C₂H₅ | | |
| 467 | CH₂=CH-O | *n*-C₃H₇ | | |
| 468 | CH₂=CH-O | *n*-C₄H₉ | | |
| 469 | CH₂=CH-O | *n*-C₅H₁₁ | | |
| 470 | CH₂=CH-O | *n*-C₆H₁₃ | | |
| 471 | CH₂=CH-O | *n*-C₇H₁₅ | | |
| 472 | CH₂=CH-O | CH₃O | | |
| 473 | CH₂=CH-O | C₂H₅O | | |
| 474 | CH₂=CH-O | *n*-C₃H₇O | | |
| 475 | CH₂=C H-O | *n*-C₄H₉O | | |
| 476 | CH₂=CH-O | CH₂=CH | | |
| 477 | CH₂=CH-O | *E*-CH₃-CH=CH | | |
| 478 | CH₂=CH-O | CH₂=CH-O | | |
| 479 | CH₂=CH-O | CH₂=CH-CH₂O | | |

### Beispiele 480 bis 509

Analog zu Beispiel 1.4 werden hergestellt: 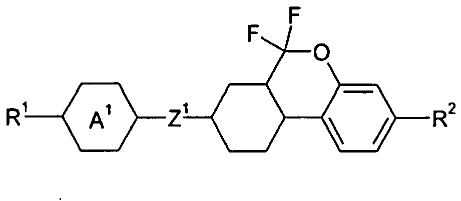 worin 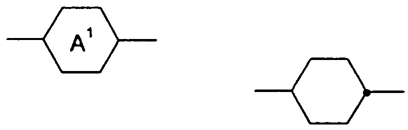 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 480 | CH₃ | CH₃ | | |
| 481 | CH₃ | C₂Hs | | |
| 482 | CH₃ | *n*-C₃H₇ | | |
| 483 | C₂H₅ | CH₃ | | |
| 484 | C₂H₅ | C₂H₅ | | |
| 485 | C₂H₅ | *n*-C₃H₇ | | |
| 486 | *n*-C₃H₇ | CH₃ | | |
| 487 | *n*-C₃H₇ | C₂H₅ | | |
| 488 | *n*-C₃H₇ | *n*-C₃H₇ | | |
| 489 | *n*-C₃H₇ | *n*-C₅H₁₁ | | |
| 490 | *n*-C_{S}H₁1 | *n*-C₃H₇ | | |
| 491 | *n*-C₅H₁₁ | *n*-C₅H₁₁ | | |
| 492 | CH₂=CH | CH₃ | | |
| 493 | CH₂=CH | C₂H₅ | | |
| 494 | CH₂=CH | *n*-C₃H₇ | | |
| 495 | CH₂=CH | CH₂=CH | | |
| 496 | CH₃ | CH₂=CH | | |
| 497 | C₂H₅ | CH₂=CH | | |
| 498 | *n*-C₃H₇ | CH₂=CH | | |
| 499 | *E*-CH₃-CH=CH | CH₂=CH | | |
| 500 | *E*-CH₃-CH=CH | *E*-CH₃-CH=CH | | |
| 501 | CH₃ | CH₃O | | |
| 502 | CH₃ | C₂H₅O | | |
| 503 | CH₃ | *n*-C₃H₇O | | |
| 504 | *n*-C₃H₇ | CH₃O | | |
| 505 | *n*-C₃H₇ | C₂H₅O | | |
| 586 | *n*-C₃H₇ | *n*-C₃H₇O | | |
| 507 | CH₃O | CH₃O | | |
| 508 | C₂H₅O | C₂H₅O | | |
| 509 | *n*-C₃H₇O | *n*-C₃H₇O | | |

### Beispiele 510 bis 539

Analog zu Beispiel 1.4. werden hergestellt: 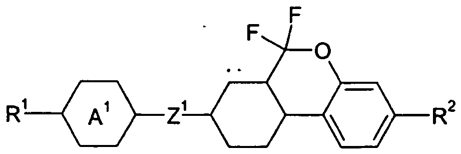 worin 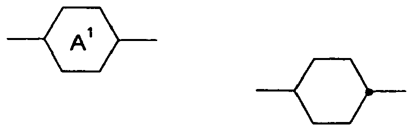 und
- Z¹: -CH₂-CH₂-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 510 | CH₃ | CH₃ | | |
| 511 | CH₃ | C₂H₅ | | |
| 512 | CH₃ | *n*-C₃H₇ | | |
| 513 | C₂H₅ | CH₃ | | |
| 514 | C₂H₅ | C₂H₅ | | |
| 515 | C₂H₅ | *n*-C₃H₇ | | |
| 516 | *n*-C₃H₇ | CH₃ | | |
| 517 | *n*-C₃H₇ | C₂H₅ | | |
| 518 | *n*-C₃H₇ | *n*-C₃H₇ | | |
| 519 | *n*-C₃H₇ | *n*-C₅H₁₁ | | |
| 520 | *n*-C₅H₁₁ | *n*-C₃H₇ | | |
| 521 | *n*-C₅H₁₁ | *n*-C₅H₁₁ | | |
| 522 | CH₂=CH | CH₃ | | |
| 523 | CH₂=CH | C₂H₅ | | |
| 524 | CH₂=CH | *n*-C₃H₇ | | |
| 525 | CH₂=CH | CH₂=CH | | |
| 526 | CH₃ | CH₂=CH | | |
| 527 | C₂H₅ | CH₂=CH | | |
| 528 | *n*-C₃H₇ | CH₂=CH | | |
| 529 | *E*-CH₃-CH=CH | CH₂=CH | | |
| 530 | *E*-CH₃-CH=CH | E-CH₃-CH=CH | | |
| 531 | CH₃ | CH₃O | | |
| 532 | CH₃ | C₂H₅O | | |
| 533 | CH₃ | *n*-C₃H₇O | | |
| 534 | *n*-C₃H₇ | CH₃O | | |
| 535 | *n*-C₃H₇ | C₂H₅O | | |
| 536 | *n*-C₃H₇ | *n*-C₃H₇O | | |
| 537 | CH₃O | CH₃O | | |
| 538 | C₂H₅O | C₂H₅O | | |
| 539 | *n*-C₃H₇O | *n*-C₃H₇O | | |

### Beispiele 540 bis 569

Analog zu Beispiel 2.2. werden hergestellt: 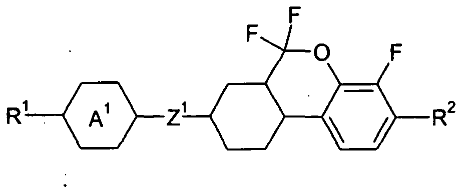 worin 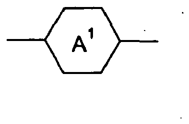 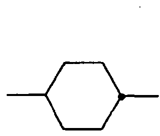 und
- Z¹: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 540 | CH₃ | CH₃ | | |
| 541 | CH₃ | C₂H₅ | | |
| 542 | CH₃ | *n*-C₃H₇ | | |
| 543 | C₂H₅ | CH₃ | | |
| 544 | C₂H₅ | C₂H₅ | | |
| 545_ | C₂H₅ | *n*-C₃H₇ | | |
| 546 | *n*-C₃H₇ | CH₃ | | |
| 547 | *n*-C₃H₇ | C₂H₅ | | |
| 548 | *n*-C₃H₇ | *n*-C₃H₇ | | |
| 549 | *n*-C₃H₇ | *n*-C₅H₁₁ | | |
| 550 | *n*-C₅H₁₁ | *n*-C₃H₇ | | |
| 551 | *n*-C₅H₁₁ | *n*-C₅H₁₁ | | |
| 552 | CH₂=CH | CH₃ | | |
| 553 | CH₂=CH | C₂H₅ | | |
| 554 | CH₂=CH | *n*-C₃H₇ | | |
| 555 | CH₂=CH | CH₂=CH | | |
| 556 | CH₃ | CH₂=CH | | |
| 557 | C₂H₅ | CH₂=CH | | |
| 558 | *n*-C₃H₇ | CH₂=CH | | |
| 559 | *E*-CH₃-CH=CH | CH₂=CH | | |
| 560 | *E*-CH₃-CH=CH | *E*-CH₃-CH=CH | | |
| 561 | CH₃ | CH₃O | | |
| 562 | CH₃ | C₂H₅O | | |
| 563 | CH₃ | *n*-C₃H₇O | | |
| 564 | *n*-C₃H₇ | CH₃O | | |
| 565 | *n*-C₃H₇ | C₂H₅O | | |
| 566 | *n*-C₃H₇ | *n*-C₃H₇O | | |
| 567 | CH₃O | CH₃O | | |
| 568 | C₂H₅O | C₂H₅O | | |
| 569 | *n*-C₃H₇O | *n*-C₃H₇O | | |

### Beispiele 570 bis 599

Analog zu Beispiel 2.2. werden hergestellt: 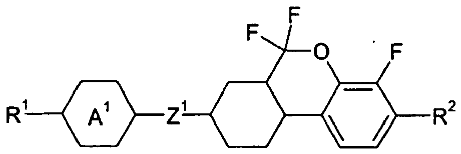 worin 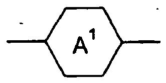 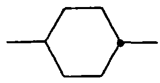 und
- Z¹: -CH₂-CH₂-
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 570 | CH₃ | CH₃ | | |
| 571 | CH₃ | C₂H₅ | | |
| 572 | CH₃ | *n*-C₃H₇ | | |
| 573 | C₂H₅ | CH₃ | | |
| 574 | C₂H₅ | C₂H₅ | | |
| 575 | C₂H₅ | *n*-C₃H₇ | | |
| 576 | *n*-C₃H₇ | CH₃ | | |
| 577 | *n*-C₃H₇ | C₂H₅ | | |
| 578 | *n*-C₃H₇ | *n*-C₃H₇ | | |
| 579 | *n*-C₃H₇ | *n*-C₅H₁₁ | | |
| 580 | *n*-C₅H₁₁ | *n*-C₃H₇ | | |
| 581 | *n*-C₅H₁₁ | *n*-C₅H₁₁ | | |
| 582 | CH₂=CH | CH₃ | | |
| 583 | CH₂=CH | C₂H₅ | | |
| 584 | CH₂=CH | *n*-C₃H₇ | | |
| 585 | CH₂=CH | CH₂=CH | | |
| 586 | CH₃ | CH₂=CH | | |
| 587 | C₂H₅ | CH₂=CH | | |
| 588 | *n*-C₃H₇ | CH₂=CH | | |
| 589 | *E*-CH₃-CH=CH | CH₂=CH | | |
| 590 | *E*-CH₃-CH=CH | *E*-CH₃-CH=CH | | |
| 591 | CH₃ | CH₃O | | |
| 592 | CH₃ | C₂H₅O | | |
| 593 | CH₃ | *n*-C₃H₇O | | |
| 594 | *n*-C₃H₇ | CH₃O | | |
| 595 | *n*-C₃H₇ | C₂H₅O | | |
| 596 | *n*-C₃H₇ | *n*-C₃H₇O | | |
| 597 | CH₃O | CH₃O | | |
| 598 | C₂H₅O | C₂H₅O | | |
| 599 | *n*-C₃H₇O | *n*-C₃H₇O | | |

### Beispiele 600 bis 629

Analog zu Beispiel 1.4. werden hergestellt: 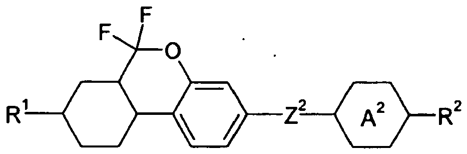 worin 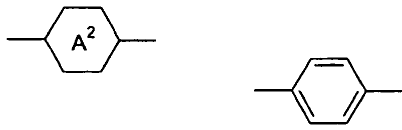 und
- Z²: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 600 | CH₃ | CH₃ | | |
| 601 | CH₃ | C₂H₅ | | |
| 602 | CH₃ | *n*-C₃H₇ | | |
| 603 | C₂H₅ ° | CH₃ | | |
| 604 | C₂H₅ | C₂H₅ | | |
| 605 | C₂H₅ | *n*-C₃H₇ | | |
| 606 | *n*-C₃H₇ | CH₃ | | |
| 607 | *n*-C₃H₇ | C₂H₅ | | |
| 608 | *n*-C₃H₇ | *n*-C₃H₇ | | |
| 609 | *n*-C₃H₇ | *n*-C₅H₁₁ | | |
| 610 | *n*-C₅H₁₁ | *n*-C₃H₇ | | |
| 611 | *n*-C₅H₁₁ | *n*-C₅H₁₁ | | |
| 612 | CH₂=CH | CH₃ | | |
| 613 | CH₂=CH | C₂H₅ | | |
| 614 | CH₂=CH | *n*-C₃H₇ | | |
| 615 | CH₂=CH | CH₂=CH | | |
| 616 | CH₃ | CH₂=CH | | |
| 617 | C₂H₅ | CH₂=CH | | |
| 618 | *n*-C₃H₇ | CH₂=CH | | |
| 619 | *E*-CH₃-CH=CH | CH₂=CH | | |
| 620 | *E*-CH₃-CH=CH | *E*-CH₃-CH=CH | | |
| 621 | CH₃ | CH₃O | | |
| 622 | CH₃ | C₂H₅O | | |
| 623 | CH₃ | *n*-C₃H₇O | | |
| 624 | *n*-C₃H₇ | CH₃O | | |
| 625 | *n*-C₃H₇ | C₂H₅O | | |
| 626 | *n*-C₃H₇ | *n*-C₃H₇O | | |
| 627 | CH₃O | CH₃O | | |
| 628 | C₂H₅O | C₂H₅O | | |
| 629 | *n*-C₃H₇O | *n*-C₃H₇O | | |

### Beispiele 630 bis 659

Analog zu Beispiel 1.4. werden hergestellt: 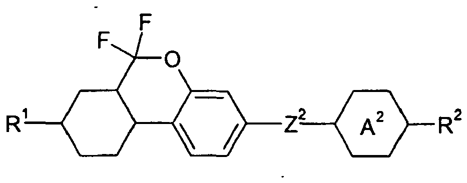 worin 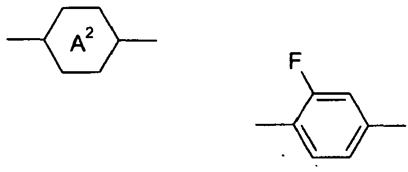 und
- Z²: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 630 | CH₃ | CH₃ | | |
| 631 | CH₃ | C₂H₅ | | |
| 632 | CH₃ | *n*-C₃H₇ | | |
| 633 | C₂H₅ | CH₃ | | |
| 634 | C₂H₅ | C₂H₅ | | |
| 635 | C₂H₅ | *n*-C₃H₇ | | |
| 636 | *n*-C₃H₇ | CH₃ | | |
| 637 | *n*-C₃H₇ | C₂H₅ | | |
| 638 | *n*-C₃H₇ | *n*-C₃H₇ | | |
| 639 | *n*-C₃H₇ | *n*-C₅H₁₁ | | |
| 640 | *n*-C₅H₁₁ | *n*-C₃H₇ | | |
| 641 | *n*-C₅H₁₁ | *n*-C₅H₁₁ | | |
| 642 | CH₂=CH | CH₃ | | |
| 643 | CH₂=CH | C₂H₅ | | |
| 644 | CH₂=CH | *n*-C₃H₇ | | |
| 645 | CH₂=CH | CH₂=CH | | |
| 646 | CH₃ | CH₂=CH | | |
| 677 | C₂H₅ | CH₂=CH | | |
| 648 | *n*-C₃H₇ , | CH₂=CH | | |
| 649 | *E*-CH₃-CH=CH | CH₂=CH | | |
| 650 | *E*-CH₃-CH=CH | *E*-CH₃-CH=CH | | |
| 651 | CH₃ | CH₃O | | |
| 652 | CH₃ | C₂H₅O | | |
| 653 | CH₃ | *n*-C₃H₇O | | |
| 654 | *n*-C₃H₇ | CH₃O | | |
| 655 | *n*-C₃H₇ | C₂H₅O | | |
| 656 | *n*-C₃H₇ | *n*-C₃H₇O | | |
| 657 | CH₃O | CH₃O | | |
| 658 | C₂H₅O | C₂H₅O | | |
| 659 | *n*-C₃H₇O | *n*-C₃H₇O | | |

### Beispiele 660 bis 689

Analog zu Beispiel 1.4. werden hergestellt: 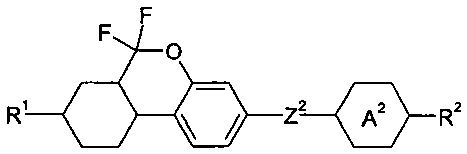 worin 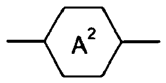 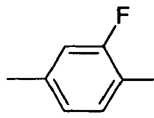 - und
- Z²: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 660 | CH₃ | CH₃ | | |
| 661 | CH₃ | C₂H₅ | | |
| 662 | CH₃ | *n*-C₃H₇ | | |
| 663 | C₂H₅ | CH₃ | | |
| 664 | C₂H₅ | C₂H₅ | | |
| 665 | C₂H₅ | *n*-C₃H₇ | | |
| 666 | *n*-C₃H₇ | CH₃ | | |
| 667 | *n*-C₃H₇ | C₂H₅ | | |
| 668 | *n*-C₃H₇ | *n*-C₃H₇ | | |
| 669 | *n*-C₃H₇ | *n*-C₅H₁₁ | | |
| 670 | *n*-C₅H11 | *n*-C₃H₇ | | |
| 671 | *n*-C₅H₁₁ | *n*-C₅H₁₁ | | |
| 672 | CH₂=CH | CH₃ | | |
| 673 | CH₂=CH | C₂H₅ | | |
| 674 | CH₂=CH | *n*-C₃H₇ | | |
| 675 | CH₂=CH | CH₂=CH | | |
| 676 | CH₃ | CH₂=CH | | |
| 677 | C₂H₅ | CH₂=CH | | |
| 678 | *n*-C₃H₇ | CH₂=CH | | |
| 679 | *E*-CH₃-CH=CH | CH₂=CH | | |
| 680 | *E*-CH₃-CH=CH | *E*-CH₃-CH=CH | | |
| 681 | CH₃ | CH₃O | | |
| 682 | CH₃ | C₂H₅O | | |
| 683 | CH₃ | *n*-C₃H₇O | | |
| 684 | *n*-C₃H₇ | CH₃O | | |
| 685 | *n*-C₃H₇ | C₂H₅O | | |
| 686 | *n*-C₃H₇ | *n*-C₃H₇O | | |
| 687 | CH₃O | CH₃O | | |
| 688 | C₂H₅O | C₂H₅O | | |
| 689 | *n*-C₃H₇O | *n*-C₃H₇O | | |

### Beispiele 690 bis 719

Analog zu Beispiel 1.4. werden hergestellt: 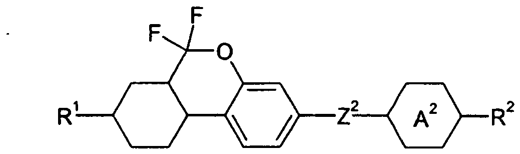 worin 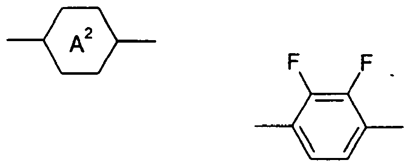 und
- Z²: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 690 | CH₃ | CH₃ | | |
| 691 | CH₃ | C₂H₅ | | |
| 692 | CH₃ | *n*-C₃H₇ | | |
| 693 | C₂H₅ | CH₃ | | |
| 694 | C₂H₅ | C₂H₅ | | |
| 695 | C₂H₅ | *n*-C₃H₇ | | |
| 696 | *n*-C₃H₇ | CH₃ | | |
| 697 | *n*-C₃H₇ | C₂H₅ | | |
| 698 | *n*-C₃H₇ | *n*-C₃H₇ | | |
| 699 | *n*-C₃H₇ | *n*-C₅H₁₁ | | |
| 700 | *n*-C₅H₁₁ | *n*-C₃H₇ | | |
| 701 | *n*-C₅H₁₁ | *n*-C₅H₁₁ | | |
| 702 | CH₂=CH | CH₃ | | |
| 703 | CH₂=CH | C₂H₅ | | |
| 704 | CH₂=CH | *n*-C₃H₇ | | |
| 705 | CH₂=CH | CH₂=CH | | |
| 706 | CH₃ | CH₂=CH | | |
| 707 | C₂H₅ | CH₂=CH | | |
| 708 | *n*-C₃H₇ | CH₂=CH | | |
| 709 | *E*-CH₃-CH=CH | CH₂=CH | | |
| 710 | *E*-CH₃-CH=CH | *E*-CH₃-CH=CH | | |
| 711 | CH₃ | CH₃0 | | |
| 712 | CH₃ | C₂H₅O | | |
| 713 | CH₃ | *n*-C₃H₇O | | |
| 714 | *n*-C₃H₇ | CH₃O | | |
| 715 | *n*-C₃H₇ | C₂H₅O | | |
| 716 | *n*-C₃H₇ | *n*-C₃H₇O | | |
| 717 | CH₃O | CH₃O | | |
| 718 | C₂H₅O | C₂H₅O | | |
| 719 | *n*-C₃H₇O | *n*-C₃H₇O | | |

### Beispiele 720 bis 749

Analog zu Beispiel 1.4. werden hergestellt: 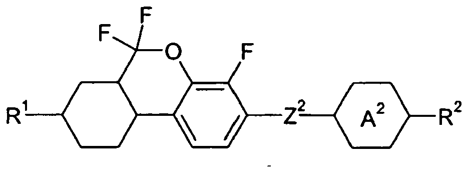 worin 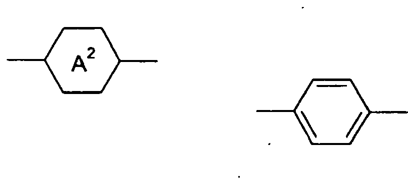 und
- Z²: eine Einfachbindung
bedeutet.

Bemerkung: * 10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 720 | CH₃ | CH₃ | | |
| 721 | CH₃ | C₂H₅ | | |
| 722 | CH₃ | *n*-C₃H₇ | | |
| 723 | C₂H₅ | CH₃ | | |
| 724 | C₂H₅ | C₂H₅ | | |
| 725 | C₂H₅ | *n*-C₃H₇ | | |
| 726 | *n*-C₃H₇ | CH₃ | | |
| 727 | *n*-C₃H₇ | C₂H₅ | | |
| 728 | *n*-C₃H₇ | *n*-C₃H₇ | | |
| 729 | *n*-C₃H₇ | *n*-C₅H₁₁ | | |
| 730 | *n*-C₅H₁₁ | *n*-C₃H₇ | | |
| 731 | *n*-C₅H₁₁ | *n*-C₅H₁₁ | | |
| 732 | CH₂=CH | CH₃ | | |
| 733 | CH₂=CH | C₂H₅ | | |
| 734 | CH₂=CH | *n*-C₃H₇ | | |
| 735 | CH₂=CH | CH₂=CH | | |
| 736 | CH₃ | CH₂=CH | | |
| 737 | C₂H₅ | CH₂=CH | | |
| 738 | *n*-C₃H₇ | CH₂=CH | | |
| 739 | *E*-CH₃-CH=CH | CH₂=CH | | |
| 740 | *E*-CH₃-CH=CH | *E*-CH₃-CH=CH | | |
| 741 | CH₃ | CH₃O | | |
| 742 | CH₃ | C₂H₅O | | |
| 743 | CH₃ | *n*-C₃H₇O | | |
| 744 | *n*-C₃H₇ | CH₃O | | |
| 745 | *n*-C₃H₇ | C₂HsO | | |
| 746 | *n*-C₃H₇ | *n*-C₃H₇O | | |
| 747 | CH₃O | CH₃O | | |
| 748 | C₂H₅0 | C₂H₅O | | |
| 749 | *n*-C₃H₇O | *n*-C₃H₇O | | |

### Beispiele 750 bis 779

Analog zu Beispiel 1.4. werden hergestellt: 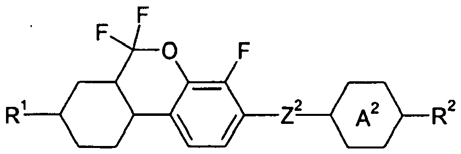 worin 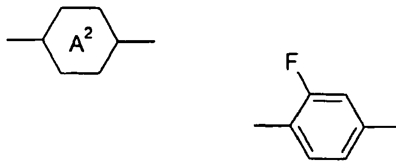 und
- Z²: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 750 | CH₃ | CH₃ | | |
| 751 | CH₃ | C₂H₅ | | |
| 752 | CH₃ | *n*-C₃H₇ | | |
| 753 | C₂H₅ | CH₃ | | |
| 754 | C₂H₅ | C₂H₅ | | |
| 755 | C₂H₅ | *n*-C₃H₇ | | |
| 756 | *n*-C₃H₇ | CH₃ | | |
| 757 | *n*-C₃H₇ | C₂H₅ | | |
| 758 | *n*-C₃H₇ | *n*-C₃H₇ | | |
| 759 | *n*-C₃H₇ | *n*-C₅H₁₁ | | |
| 760 | *n*-C₅H₁₁ | *n*-C₃H₇ | | |
| 761 | *n*-C₅H₁₁ | *n*-C₅H₁₁ | | |
| 762 | CH₂=CH | CH₃ | | |
| 763 | CH₂=CH | C₂H₅ | | |
| 764 | CH₂=CH | *n*-C₃H₇ | | |
| 765 | CH₂=CH | CH₂=CH | | |
| 766 | CH₃ | CH₂=CH | | |
| 767 | C₂H₅ | CH₂=CH | | |
| 768 | *n*-C₃H₇ | CH₂=CH | | |
| 769 | *E*-CH₃-CH=CH | CH₂=CH | | |
| 770 | *E*-CH₃-CH=CH | *E*-CH₃-CH=CH | | |
| 771 | CH₃ | CH₃O | | |
| 772 | CH₃ | C₂H₅O | | |
| 773 | CH₃ | *n*-C₃H₇O | | |
| 774 | *n*-C₃H₇ | CH₃O | | |
| 775 | *n*-C₃H₇ | C₂H₅O | | |
| 776 | *n*-C₃H₇ | *n*-C₃H₇O | | |
| 777 | CH₃O | CH₃O | | |
| 778 | C₂H₅O | C₂H₅O | | |
| 779 | *n*-C₃H₇O | *n*-C₃H₇O | | |

### Beispiele 780 bis 809

Analog zu Beispiel 1.4. werden hergestellt: 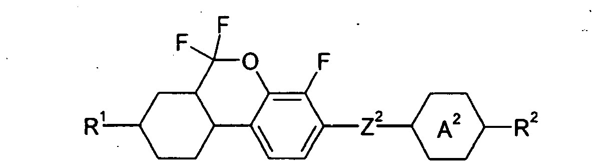 worin 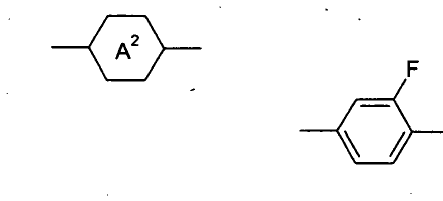 und
- Z²: eine Einfachbindung
bedeutet.

Bemerkung: *10%-iger Lösung in ZLI-4792, bzw. ZLI-2857 (Δε) extrapolierte Werte.

| Nr. | R¹ | R² | Phasensequenz T/°C | Δε* |
|---|---|---|---|---|
| 780 | CH₃ | CH₃ | | |
| 781 | CH₃ | C₂H₅ | | |
| 782 | CH₃ | *n*-C₃H₇ | | |
| 783 | C₂H₅ | CH₃ | | |
| 784 | C₂H₅ | C₂H₅ | | |
| 785 | C₂H₅ | *n*-C₃H₇ | | |
| 786 | *n*-C₃H₇ | CH₃ | | |
| 787 | *n*-C₃H₇ | C₂H₅ | | |
| 788 | *n*-C₃H₇ | *n*-C₃H₇ | | |
| 789 | *n*-C₃H₇ | *n*-C₅H₁₁ | | |
| 790 | *n*-C₅H₁₁ | *n*-C₃H₇ | | |
| 791 | *n*-C₅H₁₁ | *n*-C₅H₁₁ | | |
| 792 | CH₂=CH | CH₃ | | |
| 793 | CH₂=CH | C₂H₅ | | |
| 794 | CH₂=CH | *n*-C₃H₇ | | |
| 795 | CH₂=CH | CH₂=CH | | |
| 796 | CH₃ | CH₂=CH | | |
| 797 | C₂H₅ | CH₂=CH | | |
| 798 | *n*-C₃H₇ | CH₂=CH | | |
| 799 | *E*-CH₃-CH=CH | CH₂=CH | | |
| 800 | *E*-CH₃-CH=CH | *E*-CH₃-CH=CH | | |
| 801 | CH₃ | CH₃O | | |
| 802 | CH₃ | C₂H₅O | | |
| 803 | CH₃ | *n*-C₃H₇O | | |
| 804 | *n*-C₃H₇ | CH₃O | | |
| 805 | *n*-C₃H₇ | C₂H₅O | | |
| 806 | *n*-C₃H₇ | *n*-C₃H₇O | | |
| 807 | CH₃O | CH₃O | | |
| 808 | C₂H₅O | C₂H₅O | | |
| 809 | *n*-C₃H₇O | *n*-C₃H₇O | | |

### Beispiele 810 bis 839

Analog zu Beispiel 1.4. werden hergestellt: 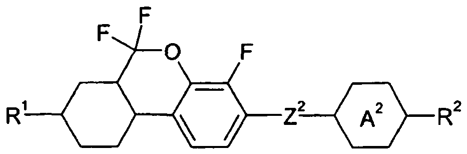 worin 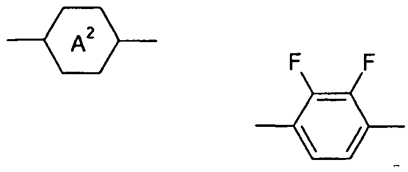 und
- Z²: eine Einfachbindung
bedeutet.

Bemerkung: * 10%-iger Lösung in ZLI-4792, bzw. ZLI-2857 (Δε) extrapolierte Werte.

| Nr: | R¹ | R² | Phasensequenz T/°C | Δε* |
|---|---|---|---|---|
| 810 | CH₃ | CH₃ | | |
| 811 | CH₃ | C₂H₅ | | |
| 812 | CH₃ | *n*-C₃H₇ | | |
| 813 | C₂H₅ | CH₃ | | |
| 814 | C₂H₅ | C₂H₅ | | |
| 815 | C₂H₅ | *n*-C₃H₇ | | |
| 816 | *n*-C₃H₇ | CH₃ | | |
| 817 | *n*-C₃H₇ | C₂H₅ | | |
| 818 | *n*-C₃H₇ | *n*-C₃H₇ | | |
| 819 | *n*-C₃H₇ | *n*-C₅H₁₁ | | |
| 820 | *n*-C₅H₁₁ | *n*-C₃H₇ | | |
| 821 | *n*-C₅H₁₁ | *n*-C₅H₁₁ | | |
| 822 | CH₂=CH | CH₃ | | |
| 823 | CH₂=CH | C₂H₅ | | |
| 824 | CH₂=CH | *n*-C₃H₇ | | |
| 825 | CH₂=CH | CH₂=CH | | |
| 826 | CH₃ | CH₂=CH | | |
| 827 | C₂H₅ | CH₂=CH | | |
| 828 | *n*-C₃H₇ | CH₂=CH | | |
| 829 | *E*-CH₃-CH=CH | CH₂=CH | | |
| 830 | *E*-CH₃-CH=CH | *E*-CH₃-CH=CH | | |
| 831 | CH₃ | CH₃O | | |
| 832 | CH₃ | C₂H₅O | | |
| 833 | CH₃ | *n*-C₃H₇O | | |
| 834 | *n*-C₃H₇ | CH₃O | | |
| 835 | *n*-C₃H₇ | C₂H₅O | | |
| 836 | *n*-C₃H₇ | *n*-C₃H₇O | | |
| 837 | CH₃O | CH₃O | | |
| 838 | C₂H₅O | C₂H₅O | | |
| 839 | *n*-C₃H₇O | *n*-C₃H₇O | | |

### Mischungsbeispiele

Es werden flüssigkristalline Gemische hergestellt und auf ihre anwendungstechnischen Eigenschaften untersucht.

### Beispiel M 1

Es wurde eine Flüssigkristallmischung mit der in der folgenden Tabelle angegebenen Zusammensetzung hergestellt und untersucht. Sie hat die ebenfalls in der Tabelle gezeigten Eigenschaften.

| Zusammensetzung | | | Physikalische Eigenschaften | |
|---|---|---|---|---|
| Verbindung | | Konz. | T(N,1) = | 86 °C |
| # | Abkürzung | /Massen-% | | |
| 1 | CY-3-04 | 7 | nₑ(20°C,589nm) = | 1.6161 |
| 2 | CY-5-02 | 5 | Δn(20°C,589nm) = | 0.1207 |
| 3 | CCY-3-02 | 7 | | |
| 4 | CCY-4-02 | 8 | ε_{⊥}(20°C, 1 kHz) = | 7.3 |
| 5 | CCY-3-03 | 7 | Δε(20°C, 1 kHz) = | -4.2 |
| 6 | CPY-2-02 | 9 | | |
| 7 | CPY-3-02 | 9 | | |
| 8 | PYP-2-3 | 8 | | |
| 9 | PYP-2-4 | 8 | | |
| 10 | CC-5-V | 9 | | |
| 11 | CC-4-V | 6 | | |
| 12 | CC-3-V1 | 6 | | |
| 13 | CCH-301 | 6 | | |
| 14 | ***Verb. 398*** | 5 | | |
| Σ | | 100,0 | | |

Das Flüssigkristallmedium hat sehr gute anwendungstechnische Eigenschaften und kann für verschiedene VA-Technologien wie MVA, PVA, ASV und auch für IPS eingesetzt werden.

## Patentansprüche

1. Verbindung der Formel I 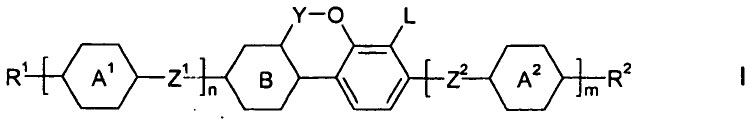 worin
Y -CF₂-,
L H, Halogen oder -CF₃, 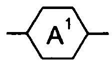
und 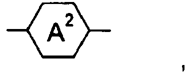 jeweils unabhängig voneinander, und wenn mehrfach vorhanden auch diese unabhängig voneinander,
(a) einen trans-1,4-Cyclohexylenrest, worin auch eine oder zwei nicht benachbarte CH₂-Gruppen durch -O- und/oder -S- ersetzt sein können,
(b) einen 1,4-Cyclohexenylenrest,
(c) einen 1,4-Phenylenrest, worin auch eine oder zwei nicht benachbarte CH-Gruppen durch N ersetzt sein können oder
(d) Naphtalin-2,6-diyl, Decahydronaphthalin-2,6-diyl und 1,2,3,4-Tetrahydronaphthalin-2,6-diyl,
(e) einen Rest ausgewählt aus der Gruppe 1,4-Bicyclo-[2,2,2]-octylen, 1,3-Bicyclo-[1,1,1]-pentylen, Spiro-[3,3]-heptan-2,6-diyl
wobei in
(a) und (b) eine oder mehrere -CH₂- Gruppen, unabhängig voneinander, jeweils durch eine -CHF- oder eine -CF₂- Gruppe ersetzt sein können und in
(c) und (d) eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe ersetzt sein können, 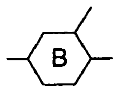 einen (1,4)-trans-Cyclohexan 1,2,4-triylrest, worin eine oder mehrere -CH₂- Gruppen, jeweils unabhängig voneinander, jeweils durch eine -CHF-oder eine -CF₂- Gruppe ersetzt sein können und die -CH< Gruppe durch eine -CF< Gruppe ersetzt sein kann, und die optional eine C-C Doppelbindung enthalten kann wobei in diesem Fall eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe ersetzt sein können,
R¹ und R² , jeweils unabhängig voneinander, H, Halogen, -CN, -SCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 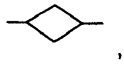 -CO-, -CO-O-, -O-CO- oder -O-CO-O- so ersetzt sein können, dass weder O- noch S-Atome direkt miteinander verknüpft sind,
Z¹ und Z² , jeweils unabhängig voneinander, -CH₂-CH₂-, -CF₂-CF₂-, -CF₂-CH₂-,-CH₂-CF₂-, -CH=CH-, -CF=CF-, -CF=CH-, -CH=CF-, -C≡C-, -COO-, -OCO-, -CH₂O-, -CH₂O-, -CF₂O-, -OCF₂-, oder eine Kombination von zweien dieser Gruppen, wobei keine zwei O-Atome miteinander verbunden sind und
n und m jeweils 0, 1 oder 2, wobei
n + m 0,1, 2 oder 3,
bedeuten.

2. Verbindung der Formeln 1 nach Anspruch 1, der Formel I-3 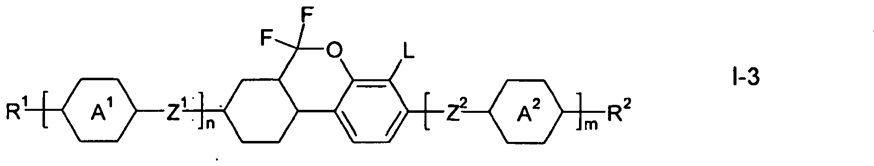 worin die Parameter die in Anspruch 1 gegebene Bedeutung haben.

3. Verbindung nach mindestens einem der Ansprüche 1 und 2, **dadurch gekennzeichnet, dass** L F bedeutet.

4. Verbindung nach mindestens einem der Ansprüche 1 bis 3, **dadurch gekennzeichnet, dass** Z¹ und Z² beide eine Einfachbindung bedeuten.

5. Flüssigkristallmedium, **dadurch gekennzeichnet, dass** es eine oder mehrere Verbindungen der Formel 1, wie in mindestens einem der Ansprüche 1 bis 4 definiert, enthält.

6. Flüssigkristallmedium, **dadurch gekennzeichnet, dass** es eine nematische Phase aufweist und eine oder mehrere Verbindungen der Formel I, wie in mindestens einem der Ansprüche 1 bis 4 definiert, enthält.

7. Flüssigkristallmedium nach mindestens einem der Ansprüche 5 und 6, **dadurch gekennzeichnet, dass** es
eine oder mehrere dielektrisch negative Verbindung(en) der Formel II 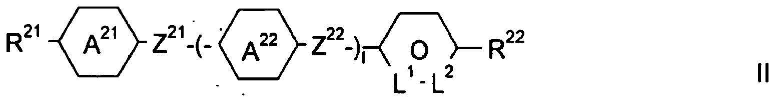 worin
R²¹ und R²² jeweils unabhängig voneinander die in Anspruch 1 bei Formel I für R¹¹ gegebene Bedeutung haben,
Z²¹ und Z²² jeweils unabhängig voneinander die in Anspruch 1 bei Formel I für Z¹¹ gegebene Bedeutung haben, 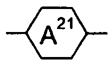
und 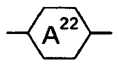 jeweils unabhängig voneinander 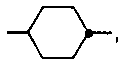 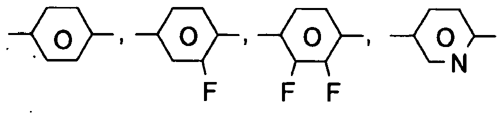 oder 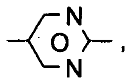
L¹ und L² beide C-F oder eines von beiden N und das andere C-F und
I 0 oder 1
bedeuten, enthält.

8. Flüssigkristallmedium nach mindestens einem der Ansprüche 5 bis 7, **dadurch gekennzeichnet, daß** es eine oder mehrere Verbindung der Formel II-1 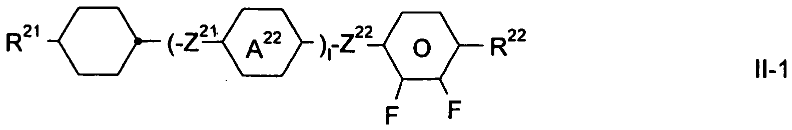 worin R²¹, R²², Z¹², Z²², 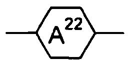 und I die in Anspruch 7
gegebene Bedeutung haben, enthält.

9. Verwendung eines Flüssigkristallmediums nach mindestens einem der Ansprüche 5 bis 8 in einer elektrooptischen Anzeige.

10. Elektrooptische Anzeige enthaltend ein Flüssigkristallmedium nach mindestens einem der Ansprüche 5 bis 8.

11. Anzeige nach Anspruch 10, **dadurch gekennzeichnet, daß** es sich um eine VAN LCD handelt.

## Claims

1. Compound of the formula I 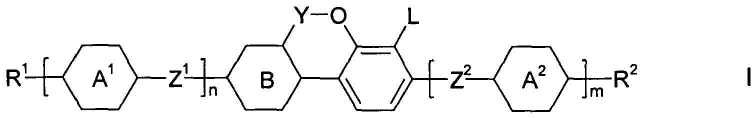 in which
Y denotes -CF₂-,
L denotes H, halogen or -CF₃, 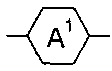
and 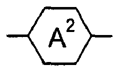 each, independently of one another and, if present more than once, also these independently of one another, denote
(a) a trans-1,4-cyclohexylene radical, in which, in addition, one or two non-adjacent CH₂ groups may be replaced by -O- and/or -S-,
(b) a 1,4-cyclohexenylene radical,
(c) a 1,4-phenylene radical, in which, in addition, one or two non-adjacent CH groups may be replaced by N, or
(d) naphthalene-2,6-diyl, decahydronaphthalene-2,6-diyl and 1,2,3,4-tetrahydronaphthalene-2,6-diyl,
(e) a radical selected from the group 1,4-bicyclo[2.2.2]-octylene, 1,3-bicyclo[1.1.1]pentylene, spiro[3.3]-heptane-2,6-diyl,
where in
(a) and (b), one or more -CH₂- groups may each be replaced, independently of one another, by a -CHF- or -CF₂- group, and in
(c) and (d), one or more -CH= groups may each be replaced, independently of one another, by a -CF=, -C(CN)=, -C(CH₃)=, -C(CH₂F)=, -C(CHF₂)=, -C(O-CH₃)=, -C(O-CHF₂)= or -C(O-CF₃)= group, 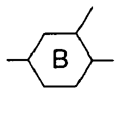 denotes a 1,4-trans-cyclohexane-1,2,4-triyl radical, in which one or more -CH₂- groups may each be replaced, in each case independently of one another, by a -CHF- or -CF₂- group, and the -CH< group may be replaced by a -CF< group, and which may optionally contain a C-C double bond, where, in this case, one or more -CH= groups may each be replaced, independently of one another, by a -CF=, -C(CN)=, -C(CH₃)=, -C(CH₂F)=, -C(CHF₂)=, -C(O-CH₃)=, -C(O-CHF₂)= or -C(O-CF₃)= group,
R¹ and R² each, independently of one another, denote H, halogen, -CN, -SCN, -SF₅, -CF₃, -CHF₂, -CH₂F, -OCF₃, -OCHF₂, an alkyl group having 1 to 15 C atoms which is monosubstituted by CN or CF₃ or at least monosubstituted by halogen, where, in addition, one or more CH₂ groups may be replaced, in each case independently of one another, by -O-, -S-, -CH=CH-, -CF=CF-, -CF=CH-, -CH=CF-, 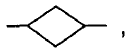 -CO-, -CO-O-, -O-CO- or -O-CO-O- in such a way that neither O nor S atoms are linked directly to one another,
Z¹ and Z² each, independently of one another, denote -CH₂-CH₂-, -CF₂-CF₂-, -CF₂-CH₂-, -CH₂-CF₂-, -CH=CH-, -CF=CF-, -CF=CH-, -CH=CF-, -C≡C-, -COO-, -OCO-, -CH₂O-, -OCH₂-, -CF₂O-, -OCF₂-, or a combination of two of these groups, where no two O atoms are bonded to one another, and
n and m each denote 0, 1 or 2, where
n + m denotes 0,1,2 or 3.

2. Compound of the formula I according to Claim 1, of the formula I-3 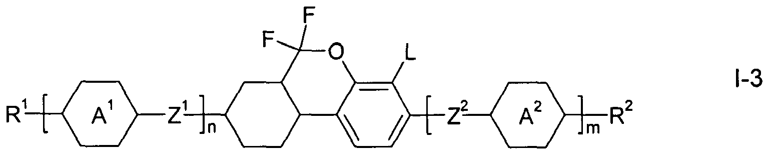 in which the parameters have the meaning given in Claim 1.

3. Compound according to at least one of Claims 1 and 2, **characterised in that** L denotes F.

4. Compound according to at least one of Claims 1 to 3, **characterised in that** Z¹ and Z² both denote a single bond.

5. Liquid-crystal medium, **characterised in that** it comprises one or more compounds of the formula I as defined in at least one of Claims 1 to 4.

6. Liquid-crystal medium, **characterised in that** it has a nematic phase and comprises one or more compounds of the formula I as defined in at least one of Claims 1 to 4.

7. Liquid-crystal medium according to at least one of Claims 5 and 6, **characterised in that** it comprises
one or more dielectrically negative compound(s) of the formula II 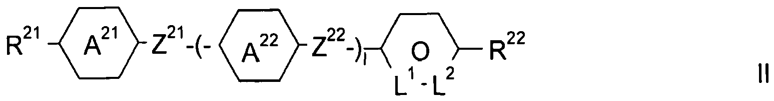 in which
R²¹ and R²² each, independently of one another, have the meaning given in Claim 1 for R¹ in the case of formula I,
Z²¹ and Z²² each, independently of one another, have the meaning given in Claim 1 for Z¹ in the case of formula I, 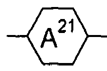
and 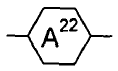 each, independently of one another, denote 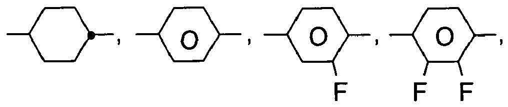 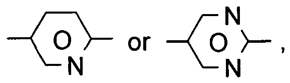
L¹ and L² both denote C-F or one of the two denotes N and the other denotes C-F, and
I denotes 0 or 1.

8. Liquid-crystal medium according to at least one of Claims 5 to 7, **characterised in that** it comprises one or more compound(s) of the formula II-1 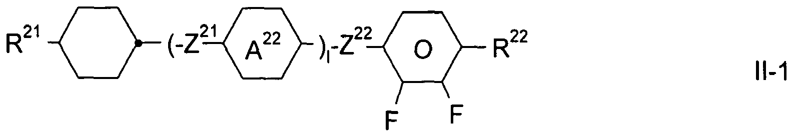 in which R²¹, R²², Z²¹, Z²², 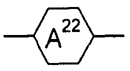 and I have the meaning given in Claim 7.

9. Use of a liquid-crystal medium according to at least one of Claims 5 to 8 in an electro-optical display.

10. Electro-optical display containing a liquid-crystal medium according to at least one of Claims 5 to 8.

11. Display according to Claim 10, **characterised in that** it is a VAN LCD.

## Revendications

1. Composé de la formule I 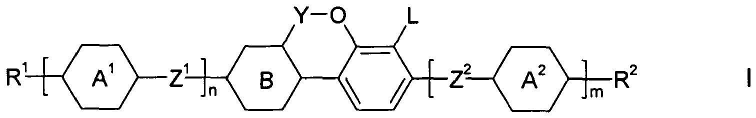 dans laquelle
Y représente -CF₂-,
L représente H, halogène ou -CF₃, 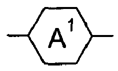
et 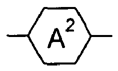 représentent, chacun indépendamment de l'autre, et dans le cas d'une occurrence supérieure à l'unité, ces occurrences représentent; également indépendamment les unes des autres,
(a) un radical trans-1,4-cyclohexylène, où, en plus, un ou deux groupes CH₂ non adjacents peut/peuvent être remplacé(s) par -O- et/ou -S-,
(b) un radical 1,4-cyclohexénylène,
(c) un radical 1,4-phénylène, où, en plus, un ou deux groupes CH non adjacents peut/peuvent être remplacé(s) par N, ou
(d) naphtalène-2,6-diyle, décahydronaphtalène-2,6-diyle et 1,2,3,4-tétrahydronaphtalène-2,6-diyle,
(e) un radical choisi parmi le groupe constitué par 1,4-bicyclo[2.2.2]octylène, 1,3-bicyclo[1.1.1]pentylène, spiro[3.3]heptane-2,6-diyle,
où, au niveau de
(a) et (b), un ou plusieurs groupes -CH₂- peuvent chacun être remplacés, indépendamment les uns des autres, par un groupe -CHF- ou -CF₂-, et au niveau de
(c) et (d), un ou plusieurs groupes -CH= peuvent chacun être remplacés, indépendamment les uns des autres, par un groupe -CF=, -C(CN)=, -C(CH₃)=, -C(CH₂F)=, -C(CHF₂)=, -C(O-CH₃)=, -C(O-CHF₂)= ou -C(O-CF₃)⁼, 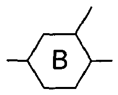
représente un radical 1,4-trans-cyclohexane-1,2,4-triyle, où un ou plusieurs groupes -CH₂- peuvent chacun être remplacés, dans chaque cas indépendamment les uns des autres, par un groupe -CHF- ou -CF₂-, et le groupe -CH< peut être remplacé par un groupe -CF<, et lequel peut en option contenir une liaison double C-C, où, dans ce cas, un ou plusieurs groupes -CH= peuvent chacun être remplacés, indépendamment les uns des autres, par un groupe -CF=, -C(CN)=, -C(CH₃)=, -C(CH₂F)=, -C(CHF₂)=, -C(O-CH₃)=, -C(O-CHF₂)= ou -C(O-CF₃)=,
R¹ et R² représentent, chacun indépendamment de l'autre, H, halogène, -CN, -SCN, -SF₅, -CF₃, -CHF₂, -CH₂F, -OCF₃, -OCHF₂, un groupe alkyle comportant de 1 à 15 atomes de C, lequel est monosubstitué par CN ou CF₃ ou au moins monosubstitué par halogène, où, en plus, un ou plusieurs groupes CH₂ peut/peuvent être remplacé(s), dans chaque cas indépendamment les uns des autres, par -O-, -S-, -CH=CH-, -CF=CF-, -CF=CH-, -CH=CF-, 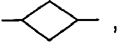 -CO-, -CO-O-, -O-CO- ou -O-CO-O- de telle sorte que ni des atomes de O, ni des atomes de S ne soient liés directement les uns aux autres,
Z¹ et Z² représentent, chacun indépendamment de l'autre, -CH₂-CH₂-, -CF₂-CF₂-, -CF₂-CH₂-, -CH₂-CF₂-, -CH=CH-, -CF=CF-, -CF=CH-, -CH=CF-, -C≡C-, -COO-, -OCO-, -CH₂O-, -OCH₂-, -CF₂O-, -OCF₂-, ou une combinaison de deux de ces groupes, où deux atomes de O ne sont jamais liés l'un à l'autre, et
n et m représentent chacun 0, 1 ou 2, où
n + m représente 0, 1, 2 ou 3.

2. Composé de la formule I selon la revendication 1, de la formule I-3 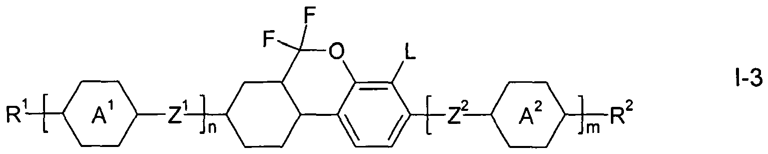 dans laquelle les paramètres présentent la signification donnée selon la revendication 1.

3. Composé selon au moins l'une des revendications 1 et 2, **caractérisé en ce que** L représente F.

4. Composé selon au moins l'une des revendications 1 à 3, **caractérisé en ce que** Z¹ et Z² représentent tous deux une liaison simple.

5. Milieu cristallin liquide, **caractérisé en ce qu'**il comprend un ou plusieurs composés de la formule I comme défini selon au moins l'une des revendications 1 à 4.

6. Milieu cristallin liquide, **caractérisé en ce qu'**il comporte une phase nématique et comprend un ou plusieurs composés de la formule I comme défini selon au moins l'une des revendications 1 à 4.

7. Milieu cristallin liquide selon au moins l'une des revendications 5 et 6, **caractérisé en ce qu'**il comprend
un ou plusieurs composé(s) diélectriquement négatif(s) de la formule II 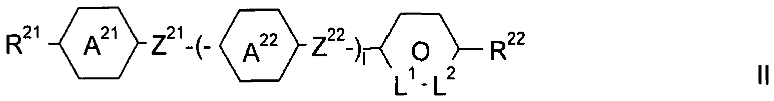 dans laquelle
R²¹ et R²² présentent, chacun indépendamment de l'autre, la signification donnée selon la revendication 1 pour R¹ dans le cas de la formule I,
Z²¹ et Z²² présentent, chacun indépendamment de l'autre, la signification donnée selon la revendication 1 pour Z¹ dans le cas de la formule I, 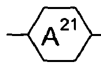
et 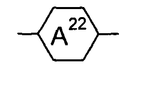 représentent, chacun indépendamment de l'autre, 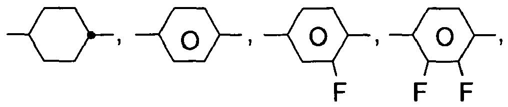 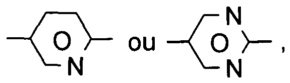
L¹ et L² représentent tous deux C-F ou l'un des deux représente N et l'autre représente C-F, et
I représente 0 ou 1.

8. Milieu cristallin liquide selon au moins l'une des revendications 5 à 7, **caractérisé en ce qu'**il comprend un ou plusieurs composé(s) de la formule II-1 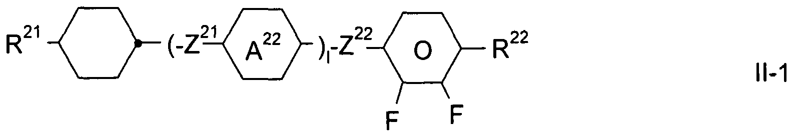 dans laquelle R²¹, R²², Z²¹, Z²², 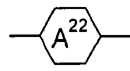 et I présentent la signification donnée selon la revendication 7.

9. Utilisation d'un milieu cristallin liquide selon au moins l'une des revendications 5 à 8 dans un affichage électro-optique.

10. Affichage électro-optique contenant un milieu cristallin liquide selon au moins l'une des revendications 5 à 8.

11. Affichage selon la revendication 10, **caractérisé en ce qu'**il s'agit d'un VAN LCD.
